# EUROPÄISCHE PATENTANMELDUNG

(11) **EP 3 002 335 A1**
(43) Veröffentlichungstag der Anmeldung: **06.04.2016**
(21) Anmeldenummer: 14187583.1
(22) Anmeldetag: 03.10.2014
(51) Int. Cl.: C12P 17/06, C12N 9/02, C12N 9/10

(54) **Verfahren zur biotechnologischen Herstellung von Flavonoiden**

(71) Anmelder: Symrise AG, 37603 Holzminden (DE)
(72) Erfinder: Wessjohann, Ludger, 06120 Halle (Saale) (DE); Bauer, Anne-Katrin, 06132 Halle (Saale) (DE); Ley, Jakob, 37603 Holzminden (DE); Geißler, Torsten, 37574 Einbeck (DE); Geißler, Katrin, 37574 Einbeck (DE)
(74) Vertreter: Fabry, Bernd

(57) **Zusammenfassung**

Vorgeschlagen wird ein Verfahren zur Herstellung von Flavonoiden, umfassend die Schritte:
(a) Bereitstellen eines transgenen Mikroorganismus, enthaltend
(i) einen ersten Nukleinsäure-Abschnitt (A), umfassend oder bestehend aus einem für eine CYP450-Oxidase kodierenden Gen,
(ii) einen zweiten Nukleinsäure-Abschnitt (B), umfassend oder bestehend aus einem für eine O-Methyltransferase kodierenden Gen, sowie
(b) Hinzufügen eines oder mehrerer Flavanone zu dem transgenen Mikroorganismus,
(c) Kultivierung des transgenen Mikroorganismus unter Bedingungen, die die Umsetzung der Flavanone zu den entsprechenden Flavonoiden ermöglichen, sowie gegebenenfalls
(d) Isolierung und Aufreinigung der Endprodukte.

## Beschreibung

### GEBIET DER ERFINDUNG

Die Erfindung befindet sich auf dem Gebiet der Biotechnologie und betrifft ein Verfahren, bei dem man Flavanone ohne chemische Zwischenschritte in die entsprechenden Flavonoide umwandelt, einen entsprechenden Mikroorganismus, einen Vektor und eine Wirtszelle.

### STAND DER TECHNIK

Flavonoide wie beispielsweise Homoeriodictyol, Eriodictyol (EP 1258200 B1) und Hesperetin (EP 1998636 B1) stellen wichtige Geschmacks- und Aromastoffe dar. Üblicherweise werden diese Stoffe durch Aldolreaktion von freiem oder partiell methylierten Acetophloroglucinol mit freiem oder partiell methylierten Protocatechualdehyd erzeugt. Obwohl die Verfahren inzwischen industriell etabliert sind, weisen sie entscheidende Nachteile auf: so besteht die Notwendigkeit, die phenolischen Funktionen während des Reaktionsverlaufes mit Schutzgruppen zu versehen, die später wieder entfernt werden müssen. Dadurch werden die Synthesen vielstufig, d.h. technisch aufwendig und im Ergebnis natürlich auch kostspielig.

Besonders nachteilig ist indes, dass es sich hierbei um chemische Herstellverfahren handelt, d.h. aus regulatorischen Gründen dürfen die damit herstellten Lebensmittelzusatzstoffe, Geschmacksstoffe und Aromen nicht als natürlich bezeichnet werden. Die Kennzeichnung als natürlich oder naturidentisch ist indes für viele Verbraucher für ausschlaggebend für eine Kaufentscheidung, es liegt daher auf der Hand, dass ein besonderer Bedarf an entsprechenden Flavonoiden besteht, die diese Kennzeichnung tragen dürfen.

Ein Ausweg könnte die Gewinnung der Flavonoiden aus pflanzlichen Rohstoffen sein, beispielsweise durch Extraktion aus *Eriodictyon spp.* Diese Prozesse sind jedoch zeit- und kostenaufwändig, beruhen zum Teil auf Wildsammlungen der Pflanzen, sind saisonabhängig, Lösungsmittel- und Prozess-intensiv und daher ebenfalls für eine breite kommerzielle Nutzung ungeeignet.

Die Aufgabe der vorliegenden Erfindung hat somit darin bestanden, ein Verfahren zur Herstellung von Flavonoiden, insbesondere von Eriodictyol, Hesperetin, Sterubin und/oder Homoeriodictyol zur Verfügung zu stellen, das frei von den eingangs geschilderten Nachteilen ist. Ein besonderes Augenmerk sollte darauf liegen, dass die Flavonoide in kurzen Reaktionszeiten und in hohen Ausbeuten zur Verfügung gestellt werden können, wobei das Verfahren frei von chemischen Reaktionsschritten sein sollte, damit die Reaktionsprodukte anschließend als natürlich oder naturidentisch bezeichnet werden können.

### BESCHREIBUNG DER ERFINDUNG

Ein erster Gegenstand der Erfindung betrifft ein Verfahren zur Herstellung von Flavonoiden, umfassend die Schritte:
(a) Bereitstellen eines transgenen Mikroorganismus, enthaltend
   (i) einen ersten Nukleinsäure-Abschnitt (A), umfassend oder bestehend aus einem für eine CYP450-Oxidase kodierenden Gen,
   (ii) einen zweiten Nukleinsäure-Abschnitt (B), umfassend oder bestehend aus einem für eine O-Methlytransferase kodierenden Gen,
(b) Hinzufügen eines oder mehrerer Flavanone zu dem transgenen Mikroorganismus,
(c) Kultivierung des transgenen Mikroorganismus, vorzugsweise einen anaeroben Mikroorganismus, unter Bedingungen, die die Umsetzung der Flavanone zu den entsprechenden Flavonoiden ermöglichen, sowie gegebenenfalls
(d) Isolierung und Aufreinigung der Endprodukte.

Überraschenderweise wurde gefunden, dass durch Einbau von zwei unterschiedlichen Nukleinsäure-Abschnitten, die einmal ein für eine bakterielle CYP450 Oxidase und zum anderen ein für eine pflanzliche O-Methyltransferase kodierendes Gen enthalten in einen geeigneten Mikroorganismus, vorzugsweise einen fakultativ anaeroben Mikroorganismus ein System zur Verfügung steht, welches bei Zugabe von Flavanonen und Kultivierung die Umsetzung zum Flavonoid in kurzen Zeiten und ausgezeichneten Ausbeuten möglich macht.

Das Verfahren wird anhand des Beispiels Homoeriodictoyl aus Naringenin in dem folgenden Schema verdeutlicht:

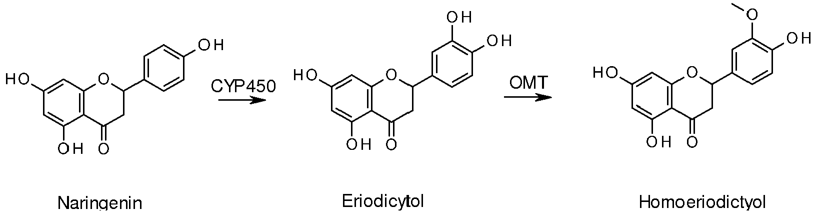

Im Stand der Technik war bekannt, dass Naringenin durch transgene Mikroorganismen wie z.B. *Sacharomyces cerevisiae,* welche eine Flavonoid-3'-hydroxylase aus *Gerbera hydrid* oder eine CYP450-Oxidase aus *Phanerochaete chrysosporium* bilden¹, in Eriodicytol umgewandelt werden kann (Kasai, N. et al. Enzymatic properties of cytochrome P450 catalyzing 3'-hydroxylation of naringenin from the white-rot fungus Phanerochaete chrysosporium. Biochem. Biophys. Res. Commun. 387, 103-8 (2009**))**. Jedoch erfolgte hier die Umwandlung nur bis zum Eriodictyol, eine direkte Weiterreaktion zum Homoeriodictyol konnte bisher nicht nachgewiesen werden.

Weitere Verfahren zur Herstellung von Eriodictyol aus Naringenin sind in Kitamura, E. et al. "Production of Hydroxlated Flavonoids with Cytochrome P450 BM3 Variant F88V and Their Antioxidative Activities" [Biosci. Biotechnol. Biochem. 77, 1340-1343 (2013**)]** und Kabumoto, H., Miyazaki, K. & Arisawa, "A Directed Evolution of the Actinomycete Cytochrome P450 MoxA (CYP105) for Enhanced Activity" [Biosci. Biotechnol. Biochem. 73, 1922-1927 (2009**)**] veröffentlicht. Weiterhin sind hierunter keine (biotechnologischen) Verfahren zur Herstellung von Eriodictyol und/oder Homoeriodictyol im Sinne der vorliegenden Erfindung zu verstehen, insbesondere kein Verfahren (wie oben beschrieben), das sich zur industriellen Herstellung von Eriodictyol und/oder Homoeriodictyol eignet, da die Ausbeuten als sehr gering einzuschätzen sind (z.B. 5,2 % in Kitamura et al.).

Im Rahmen der Untersuchungen im Zusammenhang mit der vorliegenden Erfindung konnten für die Zwecke des hierin beschriebenen Verfahrens entscheidende molekularbiologische und biochemische Grundlagen der Biotransformation - mit dem Ziel der Produktion von Eriodictyol und/oder Homoeriodictyol in industriellem Maßstab - aufgeklärt und charakterisiert werden.

Im Stand der Technik sind zwar unterschiedliche Enzymsysteme zur Bildung von Eriodictyol ausgehend von Kaffeesäure beschrieben (Leonard, E., Yan, Y. & Koffas, M. A. G. "Functional expression of a P450 flavonoid hydroxylase for the biosynthesis of plant-specific hydroxylated flavonols in Escherichia coli" [Metab. Eng. 8, 172-81 (2006**)**]. Allerdings gehen diese stets vom in Pflanzen beschriebenen Biosyntheseweg für Flavonoide aus und wobei Naringenin nur als Zwischenprodukt gebildet wird. Weiterhin werden hier ausschließlich pflanzliche Enzyme genutzt. Eine Synthesemethode zur Bildung von Homoeriodicytol oder Hesperetin ausgehend von Naringenin ist noch nicht bekannt.

In den beiden internationalen Patentanmeldungen WO 2006 010117 A1 (KOFFAS) und WO 2005 084305 A1 (SCHMIDT-DANNERT) ist die Anwendung heterologer Expression zur Bildung von Flavonoiden beschrieben. Darin werden (ausschließlich) pflanzliche Gene beschrieben, die für eine heterologe Expression verschiedener Substanzen (ausgehend von L-Phenylalanin, Tyrosin und Zimtsäure) genutzt werden.

Überraschenderweise wurde in der vorliegenden Erfindung eine Variante der CYP450-Oxidase aus *Bacillus megaterium* identifiziert, die durch heterologe Expression produziert werden konnte und Naringenin regioselektiv an der 3' bzw. 5'-Position hydroxyliert. Durch die zusätzliche Expression einer O-Methyltransferase gelingt es vorteilhafterweise, dass das intermediär gebildete Eriodictyol zum Homoeriodictyol umgewandelt wird.

### CYP450 OXIDASEN

Eine "CYP450-Oxidase" (CYP450) im Sinne der vorliegenden Erfindung ist ein Enzym, das die Reaktion "Flavanon ⇄ 3'-Hydroxyflavanon" katalysiert. Insbesondere katalysiert die CYP450 die Reaktion von Naringenin zu Eriodictyol (vgl. das eingangs gezeigte Reaktionsschema). Besonders bevorzugt sind CYP450-Oxidasen aus dem grampositiven Bakterium *Bacillus megaterium.*

Ausdrücklich bevorzugt ist daher ein Verfahren, bei dem das für eine bakterielle CYP450 Oxidase kodierende Gen für eine Oxidase aus dem Mikrorganismus *Bacillus megaterium* kodiert.

Eine besondere Ausführungsform der vorliegenden Erfindung betrifft demnach ein Verfahren, welches sich dadurch auszeichnet, dass der in den transgenen Mikroorganismus eingebrachte Nukleinsäure-Abschnitt (A)
(i-i) eine Nukleotidsequenz gemäß **SEQ ID NO:1** (Nukleotidsequenz der für die A75G/ F88V/L189Q/R472C Variante der bakterielle CYP450 aus B. *megaterium* ATCC 14581 kodierenden Gens) und/oder
(i-ii) eine Nukleotidsequenz **SEQ ID NO:2** (Nukleotidsequenz der für die A75G/ F88V/L189Q/A331S/R472C Variante der bakterielle CYP450 aus B. *megaterium* ATCC 14581 kodierenden Gens) und/oder
(i-iii) eine ähnliche Nukleotidsequenz mit einer Nukleotidsequenz-Identität von 40 % oder mehr zu **SEQ ID NO:1**und/oder **SEQ ID NO:2**,, vorzugsweise von 50 % oder mehr, 60 % oder mehr oder 80 % oder mehr, besonders bevorzugt von 95 % oder mehr,
umfasst oder daraus besteht.

*Mutatis mutandis* ist ebenfalls Gegenstand der Erfindung ein Verfahren, bei dem die vorzugsweise bakterielle CYP450 Oxidase
(i-i) eine Nukleotidsequenz gemäß **SEQ ID NO:1** (Nukleotidsequenz der für die A75G/ F88V/L189Q/R472C Variante der bakterielle CYP450 aus *B*. *megaterium* ATCC 14581 kodierenden Gens) und/oder
(i-ii) eine Nukleotidsequenz **SEQ ID NO:2** (Nukleotidsequenz der für die A75G/ F88V/L189Q/A331S/R472C Variante der bakterielle CYP450 aus *B*. *megaterium* ATCC 14581 kodierenden Gens) und/oder
(i-iii) eine ähnliche Nukleotidsequenz mit einer Nukleotidsequenz-Identität von 40 % oder mehr zu **SEQ ID NO:1** und/oder **SEQ ID NO:2**, vorzugsweise von 50 % oder mehr, 60 % oder mehr oder 80 % oder mehr, besonders bevorzugt von 95 % oder mehr,
enthält.

Eine weitere besondere Ausführungsform der vorliegenden Erfindung betrifft demnach ein Verfahren, welches sich dadurch auszeichnet, dass der in den transgenen Mikroorganismus eingebrachte Aminosäure-Abschnitt (A)
(i-i) eine Aminosäuresequenz gemäß **SEQ ID NO:6** (Aminosäuresequenz der für die A75G/ F88V/L189Q/R472C Variante der bakterielle CYP450 aus *B*. *megaterium* ATCC 14581 kodierenden Gens) und/oder
(i-ii) eine Aminosäuresequenz **SEQ ID NO:7** (Aminosäuresequenz der für die A75G/ F88V/L189Q/A331S/R472C Variante der bakterielle CYP450 aus *B*. *megaterium* ATCC 14581 kodierenden Gens) und/oder
(i-iii) eine ähnliche Aminosäuresequenz mit einer Aminosäuresequenz-Identität von 40 % oder mehr zu **SEQ ID NO:6** und/oder **SEQ ID NO:7**, vorzugsweise von 50 % oder mehr, 60 % oder mehr oder 80 % oder mehr, besonders bevorzugt von 95 % oder mehr,
umfasst oder daraus besteht.

*Mutatis mutandis* ist ebenfalls Gegenstand der Erfindung ein Verfahren, bei dem die vorzugsweise bakterielle CYP450 Oxidase
(i-i) eine Aminosäuresequenz gemäß **SEQ ID NO:6** (Aminosäuresequenz der für die A75G/ F88V/L189Q/R472C Variante der bakterielle CYP450 aus B. *megaterium* ATCC 14581 kodierenden Gens) und/oder
(i-ii) eine Aminosäuresequenz **SEQ ID NO:7** (Aminosäuresequenz der für die A75G/ F88V/L189Q/A331S/R472C Variante der bakterielle CYP450 aus B. *megaterium* ATCC 14581 kodierenden Gens) und/oder
(i-iii) eine ähnliche Aminosäuresequenz mit einer Aminosäuresequenz-Identität von 40 % oder mehr zu **SEQ ID NO:6** und/oder **SEQ ID NO:7**, vorzugsweise von 50 % oder mehr, 60 % oder mehr oder 80 % oder mehr, besonders bevorzugt von 95 % oder mehr,

enthält.Im Rahmen der vorliegenden Erfindung ist die "Nukleinsäure- und/oder Aminosäuresequenz-Identität" bevorzugt anhand des Waterman-Smith-Algorithmus mit einer Lückeneröffnungs-Strafe ("gap open penalty") von 10, einer Lückenvergrößerungsstrafe ("gap extension penalty") von 0,5 und der BLOSUM62-Matrix zu bestimmen (bezüglich des Waterman-Smith-Algorithmus siehe beispielsweise Smith, T.F. and Waterman, M.S., "Identification of common molecular subsequences", Journal of Molecular biology (1981), 147:195-197; online implementiert über die entsprechende Werkzeugseite des EMBL). Die vorgelegten Nukleotid-Sequenzen wurden mit Hilfe der Software BISSAP des Europäischen Patentamtes gemäß WIPO Standard 25 erzeugt.

### O-METHYLTRANSFERASEN

Eine "O-Methyltransferase" (OMT) im Sinne der vorliegenden Erfindung ist ein Enzym, das die regiospezifische Methylierung bestimmter Verbindungen katalysiert, insbesondere die Reaktion von Eriodicytol zu Hesperetin, Sterubin und/oder Homoeriodictyol (vgl. das eingangs gezeigte Reaktionsschema). Besonders bevorzugt sind kationunabhängige O-Methyltransferasen aus *Mesembryanthemum crystallinum* oder *Arabidopsis thaliana.*

Ausdrücklich bevorzugt ist daher ein Verfahren, bei dem das für eine pflanzliche O-Methyltransferase kodierende Gen für eine O-Methyltransferase aus der Pflanzengattung *Mesembryanthemum* kodiert.

In Summe bevorzugt sind bestimmte Kombinationen aus CYP450 Oxidasen und O-Methyltransferasen, nämlich solchen die zum einen aus dem Bakterium *Bacillis megaterium* und zum anderen aus den Pflanzen der Spezies *Mesembryanthemum crystallinum* oder *Arabidopsis thaliana* gewonnen werden.
Eine weitere besondere Ausführungsform der Erfindung betrifft daher ein Verfahren welches sich dadurch auszeichnet, dass der in den transgenen Mikroorganismus eingebrachte Nukleinsäure-Abschnitt (B)
(ii-i) eine Nukleotidsequenz gemäß **SEQ ID NO:3** (Nukleotidsequenz der OMT aus M. *crystallinum* (McPFOMT)), und/oder
(ii-ii) eine Nukleotidsequenz gemäß **SEQ ID NO:4** (Nukleotidsequenz der O-Methyltransferase 1 (OMT1)) und/oder
(ii-iii) eine Nukleotidsequenz gemäß **SEQ ID NO:5** (Nukleotidsequenz der O-Methyltransferase (AOMT)) und/oder
(ii-iv) eine weitere Nukleotidsequenz mit einer Nukleotidsequenz-Identität von 40 % oder mehr zu **SEQ ID NO:3, SEQ ID NO:4** und/oder **SEQ ID NO:5**, vorzugsweise von 50 % oder mehr, 60 % oder mehr oder 80 % oder mehr, besonders bevorzugt von 95 % oder mehr,
umfasst oder daraus besteht.

*Mutatis-mutandis* ist ebenfalls Gegenstand der Erfindung ein Verfahren, bei dem die O-Methyltransferase
(ii-i) eine Nukleotidsequenz gemäß **SEQ ID NO:3** (Nukleotidsequenz der OMT aus M. *crystallinum* (McPFOMT)), und/oder
(ii-ii) eine Nukleotidsequenz gemäß **SEQ ID NO:4** (Nukleotidsequenz der O-Methyltransferase 1 (OMT1)) und/oder
(ii-iii) eine Nukleotidsequenz gemäß **SEQ ID NO:5** (Nukleotidsequenz der O-Methyltransferase (AOMT)) und/oder
(ii-vii) eine weitere Nukleotidsequenz mit einer Nukleotidsequenz-Identität von 40 % oder mehr zu **SEQ ID NO:3, SEQ ID NO:4** und/oder **SEQ ID NO:5,** vorzugsweise von 50 % oder mehr, 60 % oder mehr oder 80 % oder mehr, besonders bevorzugt von 95 % oder mehr,
enthält.
Eine weitere besondere Ausführungsform der Erfindung betrifft daher ein Verfahren welches sich dadurch auszeichnet, dass der in den transgenen Mikroorganismus eingebrachte Aminosäure-Abschnitt (B)
(ii-i) eine Aminosäuresequenz gemäß **SEQ ID NO:8** (Aminosäuresequenz der OMT aus M. *crystallinum* (McPFOMT)), und/oder
(ii-ii) eine Aminosäuresequenz gemäß **SEQ ID NO:9** (Aminosäuresequenz der O-Methyltransferase 1 (OMT1)) und/oder
(ii-iii) eine Aminosäuresequenz gemäß **SEQ ID NO:10** (Aminosäuresequenz der O-Methyl-transferase (AOMT)) und/oder
(ii-iv) eine weitere Aminosäuresequenz mit einer Aminosäuresequenz-Identität von 40 % oder mehr zu **SEQ ID NO:8, SEQ ID NO:8** und/oder **SEQ ID NO:10**, vorzugsweise von 50 % oder mehr, 60 % oder mehr oder 80 % oder mehr, besonders bevorzugt von 95 % oder mehr,
umfasst oder daraus besteht.

*Mutatis-mutandis* ist ebenfalls Gegenstand der Erfindung ein Verfahren, bei dem die O-Methyltransferase
(ii-i) eine Aminosäuresequenz gemäß **SEQ ID NO:8** (Aminosäuresequenz der OMT aus M. *crystallinum* (McPFOMT)), und/oder
(ii-ii) eine Aminosäuresequenz gemäß **SEQ ID NO:9** (Aminosäuresequenz der O-Methyl-transferase 1 (OMT1)) und/oder
(ii-iii) eine Aminosäuresequenz gemäß **SEQ ID NO:10** (Aminosäuresequenz der O-Methyltransferase (AOMT)) und/oder
(ii-vii) eine weitere Aminosäuresequenz mit einer Aminosäuresequenz-Identität von 40 % oder mehr zu **SEQ ID NO:8, SEQ ID NO:9** und/oder **SEQ ID NO:10**, vorzugsweise von 50 % oder mehr, 60 % oder mehr oder 80 % oder mehr, besonders bevorzugt von 95 % oder mehr,

### enthält.FLAVANONE

Das bzw. ein, mehrere oder sämtliche der erfindungsgemäß zu verwendenden Flavanone bzw. Vorstufen oder Derivate davon sind vorzugsweise ausgewählt aus der Gruppe bestehend aus: Naringenin, Naringin, Narirutin, oder andere natürlich vorkommenden oder künstlich hergestellten Naringeninglycoside, Sakuranetin, Sakuranetinglycoside, Isosakuranetin, Isosakuranetinglycoside, 4',7-Dihydroxy-flavanon oder dessen Glycoside, 4',7-Dihydroxy-3'-methoxy-flavanon oder dessen Glycoside, 3',7-Dihydroxy-4'-methoxy-flavanon oder dessen Glycoside, 3',4',7-Trihydroxy-flavanon oder dessen Glycoside, wobei die Flavanone bezüglich der 2-Position des Flavanongerüsts als (S)-, als (R)-Enantiomer, als Racemat oder als beliebige Mischung der beiden Enantiomere vorliegen können.

Im Folgenden sind einige der bevorzugt einzusetzenden Flavanone exemplarisch abgebildet:

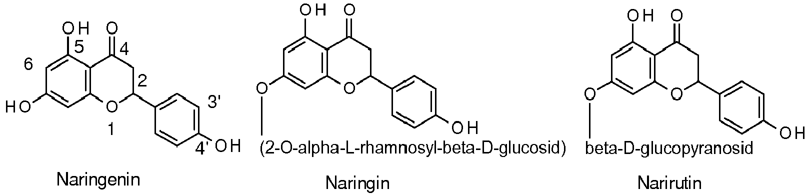

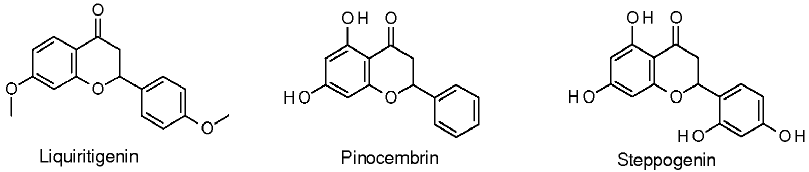

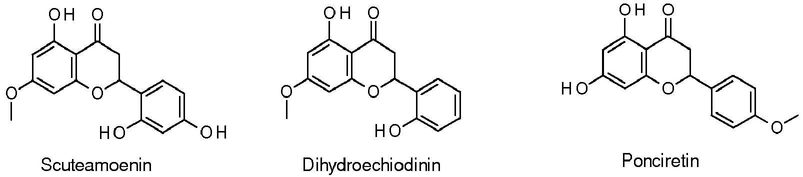

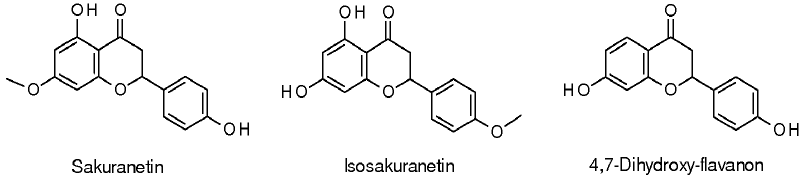

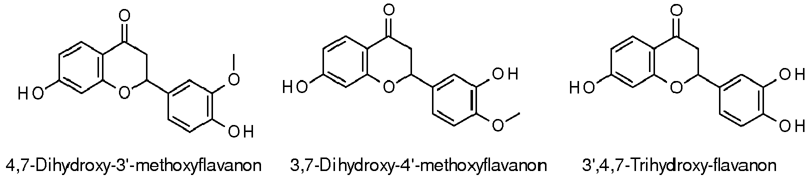

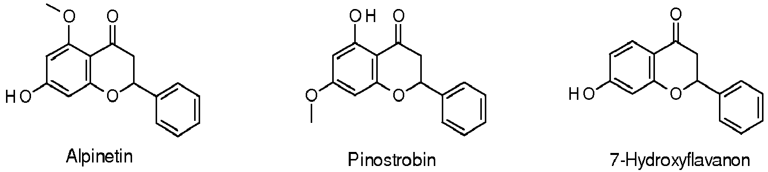

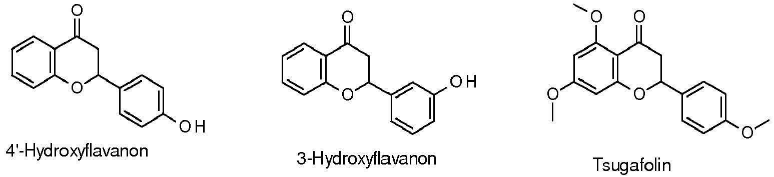

Die erfindungsgemäß herzustellenden Flavonoide sind vorzugsweise ausgewählt aus der Gruppe bestehend aus: Eriodictyol und dessen Glycoside (z.B. Eriocitrin, Neoeriocitrin), Homoeriodictyol und dessen Glycoside, Sterubin und dessen Glycoside, Hesperidin, Hesperetin und andere Hesperetinglycoside (z.B. Neohesperidin), Apigenin und dessen Glycoside, Luteolin, Diosmetin, Chrysoeriol

Im Folgenden sind erfindungsgemäß besonders bevorzugte Flavonoide abgebildet:

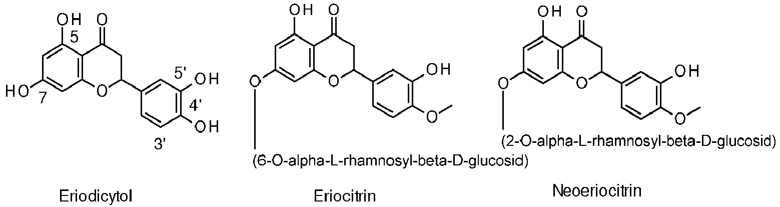

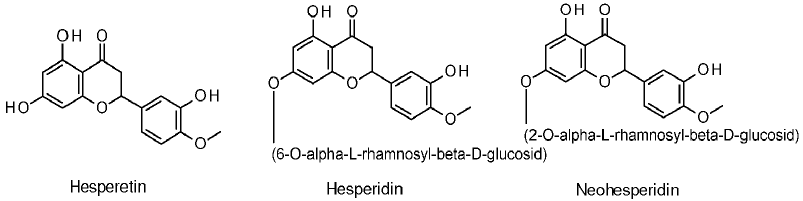

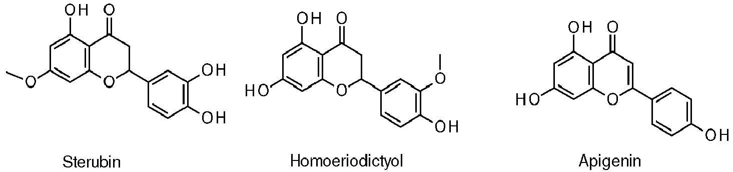

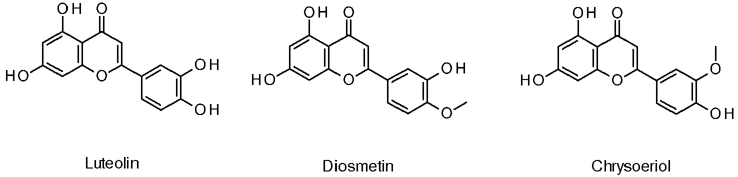

Besonders bevorzugte Flavanone und die jeweils daraus gebildeten Flavonoide sind: Naringenin und Eriodictyol, Naringenin und Homoeriodictyol, Naringenin und Sterubin, Naringenin und Hesperetin Naringin und Eriodictyol, Naringin und Homoeriodictyol, Naringin und Hesperetin, Naringenin und Apigenin, Naringenin und Luteolin, Naringenin und Sterubin, Naringenin und Diosmetin, Naringenin und Chryseriol, Naringin und Luteolin, Naringin und Sterubin, Naringin und Diosmetin sowie Naringin und Chryseriol.

### KULTIVIERUNG; EXPRESSION UND ISOLIERUNG

Wie oben beschrieben wird bzw. werden in Schritt (ii) eines erfindungsgemäßen Verfahrens ein oder mehrere Flavanone zu dem transgenen Mikroorganismus hinzugefügt, wobei der transgene Mikroorganismus unter Bedingungen kultiviert wird, die die Umsetzung des bzw. der Flavanone ermöglichen.

Gemäß einer bevorzugten Durchführung eines erfindungsgemäßen Verfahrens werden die transgenen Mikroorganismen zunächst, d.h. vor Schritt (ii), unter aeroben Bedingungen, vorzugsweise bis zum Erreichen einer maximalen Biomassenkonzentration, kultiviert. Dabei sollte die OD₆₀₀ bevorzugt wenigstens im Bereich von 1 bis 15 oder darüber liegen, vorzugsweise im Bereich von 5 bis 300, insbesondere im Bereich von 10 bis 275, bevorzugt im Bereich von 15 bis 250. Anschließend werden die Mikroorganismen in Schritt (ii) bevorzugt unter anaeroben Bedingungen kultiviert, wobei die Expression der gewünschten Aminosäuresequenzen bzw. der gewünschten Enzyme auf Basis der eingeschleusten Nukleinsäure-Abschnitte bzw. der eingeschleusten Transgene erfolgt, beispielsweise angeregt mittels Induktion durch IPTG und/oder Lactose (bei Verwendung eines entsprechenden, geeigneten Promotors bzw. eines entsprechenden, geeigneten Expressionssystems).

Grundsätzlich ist es erfindungsgemäß bevorzugt, wenn die Inkubation in Schritt (ii) zumindest teilweise oder vollständig unter aeroben Bedingungen erfolgt.

Je nach Mikroorganismus kann der Fachmann in Schritt (ii) für die Zwecke der vorliegenden Erfindung geeignete Umgebungsbedingungen schaffen und insbesondere ein geeignetes (Kultivierungs-)Medium bereitstellen. Die Kultivierung erfolgt vorzugsweise in LB-oder TB-Medium. Alternativ kann ein (komplexeres) Medium bestehend oder umfassend pflanzliche Rohstoffe, insbesondere aus Zitrus-, Grapefruit- und Orange-Pflanzen, verwendet werden. Die Kultivierung erfolgt beispielsweise bei einer Temperatur von mehr als 20 °C, bevorzugt von mehr als 25 °C, insbesondere von mehr als 30 °C (bevorzugt im Bereich von 30 bis 40 °C), was insbesondere die Bildung von Homoeriodictyol aus Naringenin begünstigen bzw. die Ausbeute erhöhen kann. Weiterhin kann auch eine Temperatur zur Induktion (vgl. oben) von weniger als 40 °C, insbesondere von weniger als 35 °C (bevorzugt im Bereich von 20 bis 30 °C), die Bildung von Homoeriodictyol begünstigen bzw. die Ausbeute erhöhen.

Die Flavonoide werden bezogen auf das (Kultivierungs-) Medium, das die transgenen Mikroorganismen enthält, in Schritt (ii) vorzugsweise in einer Menge von 0,1 mM bis 100 mM (mmol / L), bevorzugt von 0,5 bis 95 mM, besonders bevorzugt von 1 bis 90 mM, zu dem transgenen Mikroorganismus hinzugefügt. Hierbei können geeignete (Ko-)Solventien zum Einsatz kommen.

Falls zur Induktion (z.B. des lac-operons) ein oder mehrere geeignete Induktoren, z.B. IPTG oder Lactose , verwendet werden, ist es bevorzugt, den Induktor bezogen auf das (Kultivierungs-)Medium, das die transgenen Mikroorganismen enthält, in Schritt (ii) in einer Menge von 0,001 bis 1 mM, bevorzugt von 0,005 bis 0,9 mM, besonders bevorzugt von 0,01 bis 0,8 mM, einzusetzen, da hierbei besonders gute Ausbeuten erzielt werden können.

Zu Isolierung oder Aufreinigung der exprimierten Flavonoide kann beispielsweise Extraktionen mit organischen Lösemitteln vorgenommen werden. Diese sind bevorzugt ausgewählt aus der folgenden Liste: Isobutan, 2-Propanol, Toluol, Methylacetat, Cyclohexan, 2-Butanol, Hexan, 1-Propanol, Light petroleum, 1,1,1,2-Tetrafluorethan, Methanol, Propan, 1-Butanol, Butan, Ethylmethylketon, Ethylacetat, Diethylether, Ethanol, Dibutylether, CO₂, tert. Butylmethylether, Aceton, Dichloromethan und N₂O. Besonders bevorzugt sind solche Lösungsmittel, die mit Wasser eine optisch erkennbare Phasengrenze ausbilden. Hiernach kann sich eine Entfernung des Restwassers im Lösemittel sowie die Entfernung des Lösemittels selbst anbieten, an die sich wiederum ein Rücklösen des Dihydrochalkons in einem (ggf. anderen) Lösemittel anschließen kann, welches z.B. für eine gegebenenfalls nachfolgende Kristallisation und Trocknung des Produkts geeignet ist. Alternativ oder ergänzend können eine adsorptive, eine destillative und/oder eine chromatographische Aufreinigung erfolgen.

Alternativ können zur Isolierung oder Aufreinigung der gebildeten Flavonoide Verfahren der Trocknung, insbesondere Verfahren der Vakuumbandtrockung, Sprühtrocknung, Destillation oder Lyophilisation der zellhaltigen oder -freien Fermentationslösung verwendet werden.

### TRANSGENE MIKROORGANISMEN

Unter einem "transgenen Mikroorganismus" ist im Zusammenhang mit der vorliegenden Erfindung ein gentechnisch veränderter bzw. modifizierter Mikroorganismus zu verstehen, in den gezielt mittels biotechnologischer Verfahren Nukleinsäure-Abschnitte (siehe Nukleinsäure-Abschnitte (A) und (B) wie hierin beschrieben) bzw. Gene eines anderen Organismus (sog. Transgene) eingeschleust worden sind.

Ein weiterer Gegenstand der Erfindung umfasst daher einen transgener Mikroorganismus, enthaltend
(i) einen ersten Nukleinsäure-Abschnitt (A) enthaltend ein für eine bakterielle CYP450 Oxidase kodierendes Gen, sowie
(ii) einen zweiten Nukleinsäure-Abschnitt (B), enthaltend ein für eine pflanzliche O-Methyltransferase kodierendes Gen.

Vorzugsweise handelt es sich dabei um einen Mikroorganismus ausgewählt aus der Gruppe bestehend aus fakultativ anaeroben Mikroorganismen, insbesondere fakultativ anaeroben Bakterien, bevorzugt Proteobakterien, insbesondere Enterobakterien, zum Beispiel der Gattung *Escherichia,* bevorzugt *E. coli,* insbesondere *E. coli* K12, *E. coli* BL21 und *E. coli* MG1655 und deren Abkömmlinge, Hefen, zum Beispiel S. *cerevesiae* und *P. pastoris, K. lactis, H. polymorpha* sowie Pilze wie *Aspergillus spp.* oder *Trichoderma spp..*

Der erfindungsgemäße transgene Mikroorganismus zeichnet sich insbesondere dadurch aus, dass
(i) das für eine CYP450-Oxidase kodierende Gen für eine CYP450-Oxidase aus dem Mikroorganismus *Bacillus megaterium* (BM3), vorzugsweise für die F88V Variante der BM3, insbesondere für die A75G/F88V Variante der BM3, besonders bevorzugt für die Variante A75G/F88V/L189Q/R472C der BM3 oder für die Variante A75G/F88V/ L189Q/A331S/R472C der BM3 kodiert, und/oder
(ii) das für eine O-Methyltransferase kodierende Gen für eine O-Methyltransferase aus einer Pflanze der Ordnung der Brassicales, vorzugsweise der Familie der Brassicaceae, bevorzugt der Unterfamilie der Camelineae, insbesondere der Gattung der Arabidopsis, vor allem der Art *Arabidopsis thaliana,* oder der Ordnung der Caryophyllales, vorzugsweise der Familie der Aizoaceae, bevorzugt der Unterfamilie der Mesembryanthemoideae, insbesondere der Gattung der *Mesembryanthemum,* vor allem der Art *Mesembryanthemum crystalliunum,* also besonders bevorzugt für eine O-methyltransferase aus A. *thaliana* oder M. *crystalliunum* kodiert.

Methoden, um auf Basis der eingeschleusten Nukleinsäure-Abschnitte bzw. der Transgene eine Expression der gewünschten Aminosäuresequenzen bzw. der gewünschten Enzyme zu ermöglichen, sind dem Fachmann ebenfalls hinreichend bekannt, z.B. unter Verwendung eines regulatorischen Elements, insbesondere eines Promotors.

### VEKTOR

Ein weiterer Aspekt der vorliegenden Erfindung betrifft einen Vektor, d.h. ein Transportvesikel ("Genfähre") zur Übertragung von Fremd-Nukleinsäure(n) in eine Empfängerzelle, insbesondere einen Plasmidvektor, der das Klonieren eines oder mehrerer Nukleinsäure-Abschnitte ermöglicht, enthaltend
(i) einen ersten Nukleinsäure-Abschnitt (A) enthaltend ein für eine bakterielle CYP450 Oxidase kodierendes Gen, sowie
(ii) einen zweiten Nukleinsäure-Abschnitt (B), enthaltend ein für eine pflanzliche O-Methyltransferase kodierendes Gen.

Ebenfalls umfasst die Erfindung einen Vektor, vorzugsweise einen Plasmidvektor, der sich dadurch auszeichnet, dass er
(i) einen ersten Nukleinsäure-Abschnitt (A) enthaltend ein für eine bakterielle CYP450 Oxidase kodierendes Gen, sowie
(ii) einen zweiten Nukleinsäure-Abschnitt (B), enthaltend ein für eine pflanzliche O-Methyltransferase kodierendes Gen
aufweist.

Neben Nukleinsäure-Abschnitt(en) (A) und (B) kann ein erfindungsgemäßer Vektor für die Zwecke der vorliegenden Erfindung gegebenenfalls weitere übliche Bestandteile, insbesondere solche, die die Expression der hierin beschriebenen Transgene in Mikroorganismen, insbesondere in solchen wie oben beschrieben, verbessern oder gegebenenfalls erst ermöglichen. Grundsätzlich enthält ein erfindungsgemäßer Vektor vorzugsweise zudem einen oder mehrere weitere Bestandteile bzw. Elemente ausgewählt aus der Gruppe bestehend aus Promotor, ori-Sequenz, Sequenz zur affinitätschromatographischen Reinigung, Selektionsmarker, Operatorsequenz, Terminator, ribosomale Bindestellen, Proteasespaltsequenz, Rekombinationsbindestellen, Sequenzen von Fusionsproteinen und Chaperonsequenzen.

### WIRTSZELLE

Die vorliegende Erfindung betrifft auch eine Wirtszelle, enthaltend ein oder mehrere identische oder unterschiedliche erfindungsgemäße Vektoren wie hierin beschrieben. Erfindungsgemäß bevorzugt ist eine Wirtszelle, die sowohl ein oder mehrere Vektoren ....

Bei einer erfindungsgemäßen Wirtszelle handelt es sich vorzugsweise um einen erfindungsgemäß einzusetzenden bzw. einen erfindungsgemäßen Mikroorganismus (wie oben beschrieben). Die hierin beschriebenen erfindungsgemäßen Wirtszellen bzw. erfindungsgemäßen oder erfindungsgemäß einzusetzenden Mikroorganismen sind bzw. dienen vorzugsweise als (Produktions-)Stamm zur biotechnologischen Herstellung hierin beschriebener Flavonoide, insbesondere von Eriodictyol, Hesperetin, Sterubin und/oder Homoeriodictyol

### ZUBEREITUNGEN

Mit Hilfe des erfindungsgemäßen Verfahrens lassen sich somit Flavanone auf biotechnologischem Wege in Flavonoide umwandeln, die dadurch charakterisiert sind, dass sie weniger bitter und süßer schmecken als die Ausgangszubereitung und gleichzeitig als natürlich oder naturidentisch bezeichnet werden dürfen.

Ein weiterer Gegenstand der Erfindung ist daher auf Nahrungsmittelzubereitungen gerichtet, die die nach dem erfindungsgemäßen Verfahren erhaltenen Flavonoide enthalten.

Die so erhaltenen Flavanon-haltigen Lebensmittel oder zur Lebensmittelherstellung geeigneten Zubereitungen werden dann mit den erfindungsgemäßen Verfahren in Flavanoid-haltige, natürlich bezeichenbare Zubereitungen umgewandelt und können dann als solche verwendet werden oder nach Entfernung der Wirtszelle und/oder deren Bestandteilen und optionaler Aufkonzentration durch physikalische Prozesse als zur Lebensmittelherstellung geeigneten Zubereitung, bevorzugt als zur Lebensmittelherstellung geeigneten Zubereitung mit bittermaskierender oder süßverstärkender Wirkung eingesetzt werden.

Beispiele für geeignete Nahrungsmittel umfassen insbesondere Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen sowie Instantgetränke (beispielsweise Instant-Kakao-Getränke, Instant-Tee-Getränke, Instant-Kaffeegetränke, Instant-Fruchtgetränke).

Besonders bevorzugt sind dabei als Flavanon-haltigen Lebensmittel Zitrussäfte, insbesondere Orangensäfte, die einen hohen Gehalt an Naringin enthalten und durch das erfindungsgemäße Verfahren in erfindungsgemäße Flavonoid-haltige Orangensäfte umgewandelt werden können.

### BEISPIELE

### BEISPIEL 1

Bereitstellen transgener Mikroorganismen (vgl. Schritt (i))

### Transformation

Auf 50 µl aliquotierte chemisch kompetente E. coli BL21(DE) Zellen wurden für 5 Minuten auf Eis inkubiert. Nach Zugabe von 100 ng Plasmid-DNA (pSYM_BM3) wurde die Suspension gerührt und für weitere 10 Minuten auf Eis inkubiert. Die Transformation erfolgte indem die Suspension für 30 s bei 42 °C in einem Wasserbad und nachfolgend für 5 min in Eiswasser überführt wurde. Danach wurden 250 µl S.O.C. Medium zugegeben und die Zellen wurden für 1 h bei 37 °C und 200 rpm kultiviert. Letztlich wurden 200 µL der Kultur auf LB-Agar mit dem jeweiligen Antibiotikum ausgestrichen. Die Petrischale wurde für 14 h bei 37°C inkubiert.

### Mutagenese

Es wurde der QuickChange II Site-Directed Mutagenese Kit der Firma Agilent verwendet. Mit Hilfe von spezifischen Primern (Tabelle 1), welche die gewünschte Mutation samt angrenzenden unmodifizierten Bereichen enthalten, wurde eine PCR mit der BM3-Plasmid-DNA durchgeführt. Anschließend wurde der PCR-Reaktionsansatz für 2 Minuten auf Eis platziert. Danach erfolgte die Zugabe von 10 U Dpn I, wobei die Reaktion für 1 h bei 37 °C inkubiert wird. Die Primersequenzen für die Mutagenese sind in Tabelle 1 wiedergegeben.

**Tabelle 1 Primersequenzen für die Mutagenese**

| | |
|---|---|
| BM3_75G_fwd | TTGATAAAAACTTAAGTCAAGGTCTTAAATTTGTACGTGATTT |
| BM3_75G_rev | AAATCACGTACAAATTTAAGACCTTGACTTAAGTTTTTATCAA |
| BM3_189Q_fwd | TGGATGAAGCAATGAACAAGCAGCAGCGAGCAAATCCAGACGA |
| BM3_189Q_rev | TCGTCTGGATTTGCTCGCTGCTGCTTGTTCATTGCTTCATCCA |
| BM3_331S_fwd | TATGGCCAACTGCTCCTTCGTTTTCCCTAT |
| BM3_331S_rev | ATAGGGAAAACGAAGGAGCAGTTGGCCATA |

### BEISPIEL 2

Biotechnologische Herstellung von Eriodictyol (vgl. Schritt (ii))

### Enzymatische Darstellung von Eriodicytol

200 µM Naringenin wurden in 50 mM Tris-Puffer, pH 7,5 gelöst und zusammen mit 5 mM Glucose-6-phosphat, 14 U Glucose-6-phosphat dehydrogenase, 100 µM NADPH und 50 µL Zellextrakt (0,5 - 1 µM CYP450) in einem Gesamtvolumen von 1 mL für 14 h bei 25°C und 200 rpm inkubiert. Nachfolgend wurde die Reaktionslösung zweimal mit 500 µL Ethylacetat extrahiert, wobei die organischen Phasen vereinigt wurden und das Lösungsmittel im Vakuum entfernt wurde.

### Fermentative Darstellung von Eriodicytol

Mit pSYM_BM3 transformierte E. coli BL21 (DE3)-Zellen wurden in LB-Medium bis zu einer optischen Dichte bei 600 nm von 0,7 kultiviert und die Proteinproduktion durch Zugabe von 1 mM Isopropyl-β-D-thiogalactopyranosid induziert. Nach der Inkubationszeit von 3 h wurden die Kulturen direkt mit 200 µM Eriodictyol versetzt und bei 37 °C für 14 h inkubiert. Die Kulturen wurden bei 20.000 x g für 10 min zentrifugiert und mit dem dreifachen Volumen an Ethylacetat extrahiert, die organische Phase mit einer gesättigten Kochsalzlösung gewaschen und das Lösungsmittel im Vakuum abdestilliert.

### BEISPIEL 3

Biotechnologische Herstellung von Homoeriodictyol und/oder Hesperertin (vgl. Schritt (ii))

### Enzymatische Darstellung von Homoeriodicytol

20 µM Eriodicytol wurden in 50 mM Kaliumphosphatpuffer, pH 7,5 gelöst und zusammen mit 1 µM PfOMT, 250 µM Magnesiumchlorid und 1 mM S-Adenosylmethionin für 10 Minuten bei 37°C und 200 rpm inkubiert. Nachfolgend wurde die Reaktionslösung zweimal mit 500 µL Ethylacetat extrahiert, wobei die organischen Phasen vereinigt wurden und das Lösungsmittel im Vakuum entfernt wurde.

### Fermentative Darstellung von Homoeriodicytol (vgl. Schritt (ii))

Mit pSYM_BM3 und pSYM_PfOMT transformierte Zellen wurden in ZYM-5052 1:20 mit Starterkultur sowie mit 200 µM Naringenin angeimpft und bei 38°c und 200 rpm für 24 h inkubiert. Der Verlauf der optischen Dichte, der Genexpression und des Substratumsatzes wurden regelmäßige Entnahme von 1 mL Proben verfolgt. Zur Analyse wurden die Zellen durch Zentrifugation bei 14.000 x g für 10 Minuten geerntet und mittels 150 µL B-PER II-Reagenz (Thermo Scientific, Rockford) mit 50 µL DNAse I nach Herstellerangaben aufgeschlossen. Die erhaltenen Proben wurden mittels SDS-Polyacrylamidgelelektrophorese analysiert. Zur Analyse der Substratumsetzung wurden 1 mL der Kultur zweimal mit 500 µL Ethylacetat extrahiert, wobei die organischen Phasen vereinigt wurden und das Lösungsmittel im Vakuum entfernt wurde. Die Proben wurden nachfolgend in Acetonitril aufgenommen und mittels HPLC und UPLC-MS/MS analysiert.

### Enzymatische Darstellung von Hesperetin

20 µM Eriodicytol wurden in 50 mM Kaliumphosphatpuffer, pH 7,5 gelöst und zusammen mit 1 µM AOMT, 250 µM Magnesiumchlorid und 1 mM S-Adenosylmethionin für 10 Minuten bei 37°C und 200 rpm inkubiert. Nachfolgend wurde die Reaktionslösung zweimal mit 500 µL Ethylacetat extrahiert, wobei die organischen Phasen vereinigt wurden und das Lösungsmittel im Vakuum entfernt wurde.

### Fermentative Darstellung von Hesperetin

Mit pSYM_BM3 und pSYM_AOMT transformierte Zellen wurden in ZYM-5052 1:20 mit Starterkultur sowie mit 200 µM Naringenin angeimpft und bei 38°c und 200 rpm für 24 h inkubiert. Der Verlauf der optischen Dichte, der Genexpression und des Substratumsatzes wurden regelmäßige Entnahme von 1 mL Proben verfolgt. Zur Analyse wurden die Zellen durch Zentrifugation bei 14.000 x g für 10 Minuten geerntet und mittels 150 µL B-PER II-Reagenz (Thermo Scientific, Rockford) mit 50 µL DNAse I nach Herstellerangaben aufgeschlossen. Die erhaltenen Proben wurden mittels SDS-Polyacrylamidgelelektrophorese analysiert. Zur Analyse der Substratumsetzung wurde 1 mL der Kultur zweimal mit 500 µL Ethylacetat extrahiert, wobei die organischen Phasen vereinigt wurden und das Lösungsmittel im Vakuum entfernt wurde. Die Proben wurden nachfolgend in Acetonitril aufgenommen und mittels HPLC und UPLC-MS/MS analysiert.

SEQUENCE LISTING

<110> Symrise AG

<120> Verfahren zur biotechnologischen Herstellung von Flavonoiden

<130> C 3277 EP

<140> EP14187583.1
<141> 2014-10-03

<150> EP14187583.1
<151> 2014-10-03

<160> 16

<170> BiSSAP 1.3

<210> 1
<211> 3150
<212> DNA
<213> Bakterium megaterium ATCC 145811

<220>
<223> BM3 GVQ

<400> 1
atgacaatta aagaaatgcc tcagccaaaa acgtttggag agcttaaaaa tttaccgtta 60

ttaaacacag ataaaccggt tcaagctttg atgaaaattg cggatgaatt aggagaaatc 120

tttaaattcg aggcgcctgg tcgtgtaacg cgctacttat caagtcagcg tctaattaaa 180

gaagcatgcg atgaatcacg ctttgataaa aacttaagtc aaggtcttaa atttgtacgt 240

gattttgcag gagacgggtt ggttacaagc tggacgcatg aaaaaaattg gaaaaaagcg 300

cataatatct tacttccaag cttcagtcag caggcaatga aaggctatca tgcgatgatg 360

gtcgatatcg ccgtgcagct tgttcaaaag tgggagcgtc taaatgcaga tgagcatatt 420

gaagtaccgg aagacatgac acgtttaacg cttgatacaa ttggtctttg cggctttaac 480

tatcgcttta acagctttta ccgagatcag cctcatccat ttattacaag tatggtccgt 540

gcactggatg aagcaatgaa caagcagcag cgagcaaatc cagacgaccc agcttatgat 600

gaaaacaagc gccagtttca agaagatatc aaggtgatga acgacctagt agataaaatt 660

attgcagatc gcaaagcaag cggtgaacaa agcgatgatt tattaacgca tatgctaaac 720

ggaaaagatc cagaaacggg tgagccgctt gatgacgaga acattcgcta tcaaattatt 780

acattcttaa ttgcgggaca cgaaacaaca agtggtcttt tatcatttgc gctgtatttc 840

ttagtgaaaa atccacatgt attacaaaaa gcagcagaag aagcagcacg agttctagta 900

gatcctgttc caagctacaa acaagtcaaa cagcttaaat atgtcggcat ggtcttaaac 960

gaagcgctgc gcttatggcc aactgctcct gcgttttccc tatatgcaaa agaagatacg 1020

gtgcttggag gagaatatcc tttagaaaaa ggcgacgaac taatggttct gattcctcag 1080

cttcaccgtg ataaaacaat ttggggagac gatgtggaag agttccgtcc agagcgtttt 1140

gaaaatccaa gtgcgattcc gcagcatgcg tttaaaccgt ttggaaacgg tcagcgtgcg 1200

tgtatcggtc agcagttcgc tcttcatgaa gcaacgctgg tacttggtat gatgctaaaa 1260

cactttgact ttgaagatca tacaaactac gagctggata ttaaagaaac tttaacgtta 1320

aaacctgaag gctttgtggt aaaagcaaaa tcgaaaaaaa ttccgcttgg cggtattcct 1380

tcacctagca ctgaacagtc tgctaaaaaa gtatgcaaaa aggcagaaaa cgctcataat 1440

acgccgctgc ttgtgctata cggttcaaat atgggaacag ctgaaggaac ggcgcgtgat 1500

ttagcagata ttgcaatgag caaaggattt gcaccgcagg tcgcaacgct tgattcacac 1560

gccggaaatc ttccgcgcga aggagctgta ttaattgtaa cggcgtctta taacggtcat 1620

ccgcctgata acgcaaagca atttgtcgac tggttagacc aagcgtctgc tgatgaagta 1680

aaaggcgttc gctactccgt atttggatgc ggcgataaaa actgggctac tacgtatcaa 1740

aaagtgcctg cttttatcga tgaaacgctt gccgctaaag gggcagaaaa catcgctgac 1800

cgcggtgaag cagatgcaag cgacgacttt gaaggcacat atgaagaatg gcgtgaacat 1860

atgtggagtg acgtagcagc ctactttaac ctcgacattg aaaacagtga agataataaa 1920

tctactcttt cacttcaatt tgtcgacagc gccgcggata tgccgcttgc gaaaatgcac 1980

ggtgcgtttt caacgaacgt cgtagcaagc aaagaacttc aacagccagg cagtgcacga 2040

agcacgcgac atcttgaaat tgaacttcca aaagaagctt cttatcaaga aggagatcat 2100

ttaggtgtta ttcctcgcaa ctatgaagga atagtaaacc gtgtaacagc aaggttcggc 2160

ctagatgcat cacagcaaat ccgtctggaa gcagaagaag aaaaattagc tcatttgcca 2220

ctcgctaaaa cagtatccgt agaagagctt ctgcaatacg tggagcttca agatcctgtt 2280

acgcgcacgc agcttcgcgc aatggctgct aaaacggtct gcccgccgca taaagtagag 2340

cttgaagcct tgcttgaaaa gcaagcctac aaagaacaag tgctggcaaa acgtttaaca 2400

atgcttgaac tgcttgaaaa atacccggcg tgtgaaatga aattcagcga atttatcgcc 2460

cttctgccaa gcatacgccc gcgctattac tcgatttctt catcacctcg tgtcgatgaa 2520

aaacaagcaa gcatcacggt cagcgttgtc tcaggagaag cgtggagcgg atatggagaa 2580

tataaaggaa ttgcgtcgaa ctatcttgcc gagctgcaag aaggagatac gattacgtgc 2640

tttatttcca caccgcagtc agaatttacg ctgccaaaag accctgaaac gccgcttatc 2700

atggtcggac cgggaacagg cgtcgcgccg tttagaggct ttgtgcaggc gcgcaaacag 2760

ctaaaagaac aaggacagtc acttggagaa gcacatttat acttcggctg ccgttcacct 2820

catgaagact atctgtatca agaagagctt gaaaacgccc aaagcgaagg catcattacg 2880

cttcataccg ctttttctcg catgccaaat cagccgaaaa catacgttca gcacgtaatg 2940

gaacaagacg gcaagaaatt gattgaactt cttgatcaag gagcgcactt ctatatttgc 3000

ggagacggaa gccaaatggc acctgccgtt gaagcaacgc ttatgaaaag ctatgctgac 3060

gttcaccaag tgagtgaagc agacgctcgc ttatggctgc agcagctaga agaaaaaggc 3120

cgatacgcaa aagacgtgtg ggctgggtaa 3150

<210> 2
<211> 3150
<212> DNA
<213> Bakterium megaterium ATCC 145811

<220>
<223> BM3 GVQS

<400> 2
atgacaatta aagaaatgcc tcagccaaaa acgtttggag agcttaaaaa tttaccgtta 60

ttaaacacag ataaaccggt tcaagctttg atgaaaattg cggatgaatt aggagaaatc 120

tttaaattcg aggcgcctgg tcgtgtaacg cgctacttat caagtcagcg tctaattaaa 180

gaagcatgcg atgaatcacg ctttgataaa aacttaagtc aaggtcttaa atttgtacgt 240

gattttgcag gagacgggtt ggttacaagc tggacgcatg aaaaaaattg gaaaaaagcg 300

cataatatct tacttccaag cttcagtcag caggcaatga aaggctatca tgcgatgatg 360

gtcgatatcg ccgtgcagct tgttcaaaag tgggagcgtc taaatgcaga tgagcatatt 420

gaagtaccgg aagacatgac acgtttaacg cttgatacaa ttggtctttg cggctttaac 480

tatcgcttta acagctttta ccgagatcag cctcatccat ttattacaag tatggtccgt 540

gcactggatg aagcaatgaa caagcagcag cgagcaaatc cagacgaccc agcttatgat 600

gaaaacaagc gccagtttca agaagatatc aaggtgatga acgacctagt agataaaatt 660

attgcagatc gcaaagcaag cggtgaacaa agcgatgatt tattaacgca tatgctaaac 720

ggaaaagatc cagaaacggg tgagccgctt gatgacgaga acattcgcta tcaaattatt 780

acattcttaa ttgcgggaca cgaaacaaca agtggtcttt tatcatttgc gctgtatttc 840

ttagtgaaaa atccacatgt attacaaaaa gcagcagaag aagcagcacg agttctagta 900

gatcctgttc caagctacaa acaagtcaaa cagcttaaat atgtcggcat ggtcttaaac 960

gaagcgctgc gcttatggcc aactgctcct tcgttttccc tatatgcaaa agaagatacg 1020

gtgcttggag gagaatatcc tttagaaaaa ggcgacgaac taatggttct gattcctcag 1080

cttcaccgtg ataaaacaat ttggggagac gatgtggaag agttccgtcc agagcgtttt 1140

gaaaatccaa gtgcgattcc gcagcatgcg tttaaaccgt ttggaaacgg tcagcgtgcg 1200

tgtatcggtc agcagttcgc tcttcatgaa gcaacgctgg tacttggtat gatgctaaaa 1260

cactttgact ttgaagatca tacaaactac gagctggata ttaaagaaac tttaacgtta 1320

aaacctgaag gctttgtggt aaaagcaaaa tcgaaaaaaa ttccgcttgg cggtattcct 1380

tcacctagca ctgaacagtc tgctaaaaaa gtatgcaaaa aggcagaaaa cgctcataat 1440

acgccgctgc ttgtgctata cggttcaaat atgggaacag ctgaaggaac ggcgcgtgat 1500

ttagcagata ttgcaatgag caaaggattt gcaccgcagg tcgcaacgct tgattcacac 1560

gccggaaatc ttccgcgcga aggagctgta ttaattgtaa cggcgtctta taacggtcat 1620

ccgcctgata acgcaaagca atttgtcgac tggttagacc aagcgtctgc tgatgaagta 1680

aaaggcgttc gctactccgt atttggatgc ggcgataaaa actgggctac tacgtatcaa 1740

aaagtgcctg cttttatcga tgaaacgctt gccgctaaag gggcagaaaa catcgctgac 1800

cgcggtgaag cagatgcaag cgacgacttt gaaggcacat atgaagaatg gcgtgaacat 1860

atgtggagtg acgtagcagc ctactttaac ctcgacattg aaaacagtga agataataaa 1920

tctactcttt cacttcaatt tgtcgacagc gccgcggata tgccgcttgc gaaaatgcac 1980

ggtgcgtttt caacgaacgt cgtagcaagc aaagaacttc aacagccagg cagtgcacga 2040

agcacgcgac atcttgaaat tgaacttcca aaagaagctt cttatcaaga aggagatcat 2100

ttaggtgtta ttcctcgcaa ctatgaagga atagtaaacc gtgtaacagc aaggttcggc 2160

ctagatgcat cacagcaaat ccgtctggaa gcagaagaag aaaaattagc tcatttgcca 2220

ctcgctaaaa cagtatccgt agaagagctt ctgcaatacg tggagcttca agatcctgtt 2280

acgcgcacgc agcttcgcgc aatggctgct aaaacggtct gcccgccgca taaagtagag 2340

cttgaagcct tgcttgaaaa gcaagcctac aaagaacaag tgctggcaaa acgtttaaca 2400

atgcttgaac tgcttgaaaa atacccggcg tgtgaaatga aattcagcga atttatcgcc 2460

cttctgccaa gcatacgccc gcgctattac tcgatttctt catcacctcg tgtcgatgaa 2520

aaacaagcaa gcatcacggt cagcgttgtc tcaggagaag cgtggagcgg atatggagaa 2580

tataaaggaa ttgcgtcgaa ctatcttgcc gagctgcaag aaggagatac gattacgtgc 2640

tttatttcca caccgcagtc agaatttacg ctgccaaaag accctgaaac gccgcttatc 2700

atggtcggac cgggaacagg cgtcgcgccg tttagaggct ttgtgcaggc gcgcaaacag 2760

ctaaaagaac aaggacagtc acttggagaa gcacatttat acttcggctg ccgttcacct 2820

catgaagact atctgtatca agaagagctt gaaaacgccc aaagcgaagg catcattacg 2880

cttcataccg ctttttctcg catgccaaat cagccgaaaa catacgttca gcacgtaatg 2940

gaacaagacg gcaagaaatt gattgaactt cttgatcaag gagcgcactt ctatatttgc 3000

ggagacggaa gccaaatggc acctgccgtt gaagcaacgc ttatgaaaag ctatgctgac 3060

gttcaccaag tgagtgaagc agacgctcgc ttatggctgc agcagctaga agaaaaaggc 3120

cgatacgcaa aagacgtgtg ggctgggtaa 3150

<210> 3
<211> 714

<212> DNA
<213> Mesembryanthemum crystallinum

<220>
<223> McPFOMT

<400> 3
atggattttg ctgtgatgaa gcaggtcaaa aatacaggat tgctacagag tgaggagtta 60

tgccagtata ttctccgaac tagtgtctat ccgcgagaag cagggttcct caaggaactc 120

agggaagcca atgaaagtca cccagactct tatatgtcga cttcaccact tgctggacaa 180

ttgatgtcat tcgttctaaa attagtgaat gcaaagaaga ctattgaagt tggagtcttt 240

acaggatact ccctcttact cactgctctt tcaattcctg atgatggaaa gattacggca 300

attgatttcg acagagaggc atatgagatt ggcttgccat ttatcagaaa agctggtgtg 360

gagcacaaaa tcaacttcat tgaatcggat gctatgctag ctcttgacaa tcttctgcaa 420

ggacaagaga gcgaggggag ttacgacttt ggctttgttg atgcggacaa acctaactac 480

atcaagtacc atgagaggtt gatgaaacta gtcaaggtgg gtggcatagt cgcttatgac 540

aacacattat ggggtggaac tgtagcccag cctgaatccg aagtaccaga tttcatgaag 600

gaaaacagag aagctgttat tgaactcaac aagttgcttg ctgctgatcc tcgtatcgag 660

attgtacatc ttcctttggg tgatggtatc actttctgca ggcgtcttta ttga 714

<210> 4
<211> 1092
<212> DNA
<213> Mesembryanthemum crystallinum

<220>
<223> AtCOMT

<400> 4
   atgggttcaa cggcagagac acaattaact ccggtgcaag tcaccgacga cgaagctgcc 60
   ctcttcgcca tgcaactagc cagtgcttcc gttcttccga tggctttaaa atccgcctta 120
   gagcttgacc ttcttgagat tatggccaag aatggttctc ccatgtctcc taccgagatc 180
   gcttctaaac ttccgaccaa aaatcctgaa gctccggtca tgctcgaccg tatcctccgt 240
   cttcttacgt cttactccgt cttaacctgc tccaaccgta aactttccgg tgatggcgtt 300
   gaacggattt acgggcttgg tccggtttgc aagtatttga ccaagaacga agatggtgtt 360
   tccattgctg ctctttgtct tatgaaccaa gacaaggttc tcatggaaag ctggtaccat 420
   ttgaaggatg caattcttga tggtgggatt ccattcaaca aggcttatgg aatgagcgcg 480
   ttcgagtacc acgggactga ccctagattc aacaaggtct ttaacaatgg aatgtctaac 540
   cattccacaa tcaccatgaa gaagattctt gagacctata agggtttgga aggattgact 600
   tctttggttg atgttggtgg tggcattggt gctacactca aaatgattgt ctccaagtac 660
   cctaatctta aaggcatcaa ctttgatctc ccacatgtca tcgaagatgc tccttctcat 720
   cctggtattg agcatgttgg aggagatatg tttgtaagtg tccctaaagg tgatgccata 780
   ttcatgaagt ggatatgtca tgactggagt gacgaacatt gcgtgaaatt cttgaagaac 840
   tgctacgagt cacttccaga ggatggaaaa gtgatattag cagagtgtat acttccagag 900
   acaccagact caagcctctc aaccaaacaa gtagtccatg tcgattgcat tatgttggct 960
   cacaatcccg gaggcaaaga acgaaccgag aaagagtttg aggcattagc caaagcatca 1020
   ggcttcaagg gcatcaaagt tgtctgcgac gcttttggtg ttaaccttat tgagttactc 1080
   aagaagctct aa 1092

<210> 5
<211> 699
<212> DNA
<213> Mesembryanthemum crystallinum

<220>
<223> AOMT

<400> 5
atggctaagg atgaagccaa gggattgctg aaaagtgaag agctctataa atatatactg 60

gagactagtg tgtacccacg cgagccagag gttctcaggg agcttaggaa cattactcat 120

aaccaccctc aagctggtat ggctactgca ccagacgctg gccagttgat ggggatgctg 180

ctgaatctgg taaatgcaag aaagactatc gaggttgggg ttttcactgg atactctctt 240

ctcctcactg ctcttacgct accagaagat ggcaaggtta tcgccattga catgaataga 300

gactcgtatg aaataggatt gccggttata aagaaagctg gtgttgaaca caaaattgat 360

ttcaaagagt ctgaagctct tccagccctc gacgagcttt taaacaataa agtgaatgag 420

ggtggattcg acttcgcatt tgtggatgca gacaagctga attattggaa ctatcatgag 480

aggcttatta gattgatcaa agttggtggg atcatagtgt atgataacac tctctgggga 540

ggatcagttg ctgaaccgga ctcgtccacg cccgagtgga ggatagaagt caagaaagca 600

accctcgagc tgaacaagaa actctcggct gatcagcgtg tgcagatctc gcaagctgcg 660

cttggcgatg gtatcactat ttgcaggagg ttatattga 699

<210> 6
<211> 1049
<212> PRT
<213> Bakterium megaterium ATCC 145811

<220>
<223> BM3 GVQ

<400> 6
Met Thr Ile Lys Glu Met Pro Gln Pro Lys Thr Phe Gly Glu Leu Lys
1               5                   10                  15
Asn Leu Pro Leu Leu Asn Thr Asp Lys Pro Val Gln Ala Leu Met Lys

                20                  25                  30
Ile Ala Asp Glu Leu Gly Glu Ile Phe Lys Phe Glu Ala Pro Gly Arg
            35                  40                  45
Val Thr Arg Tyr Leu Ser Ser Gln Arg Leu Ile Lys Glu Ala Cys Asp
    50                  55                  60
Glu Ser Arg Phe Asp Lys Asn Leu Ser Gln Gly Leu Lys Phe Val Arg
65                  70                  75                  80
Asp Phe Ala Gly Asp Gly Leu Val Thr Ser Trp Thr His Glu Lys Asn
                85                  90                  95
Trp Lys Lys Ala His Asn Ile Leu Leu Pro Ser Phe Ser Gln Gln Ala
            100                 105                 110
Met Lys Gly Tyr His Ala Met Met Val Asp Ile Ala Val Gln Leu Val
        115                 120                 125
Gln Lys Trp Glu Arg Leu Asn Ala Asp Glu His Ile Glu Val Pro Glu
    130                 135                 140
Asp Met Thr Arg Leu Thr Leu Asp Thr Ile Gly Leu Cys Gly Phe Asn
145                 150                 155                 160
Tyr Arg Phe Asn Ser Phe Tyr Arg Asp Gln Pro His Pro Phe Ile Thr
                165                 170                 175
Ser Met Val Arg Ala Leu Asp Glu Ala Met Asn Lys Gln Gln Arg Ala
            180                 185                 190
Asn Pro Asp Asp Pro Ala Tyr Asp Glu Asn Lys Arg Gln Phe Gln Glu
        195                 200                 205
Asp Ile Lys Val Met Asn Asp Leu Val Asp Lys Ile Ile Ala Asp Arg
    210                 215                 220
Lys Ala Ser Gly Glu Gln Ser Asp Asp Leu Leu Thr His Met Leu Asn
225                 230                 235                 240
Gly Lys Asp Pro Glu Thr Gly Glu Pro Leu Asp Asp Glu Asn Ile Arg
                245                 250                 255
Tyr Gln Ile Ile Thr Phe Leu Ile Ala Gly His Glu Thr Thr Ser Gly
            260                 265                 270
Leu Leu Ser Phe Ala Leu Tyr Phe Leu Val Lys Asn Pro His Val Leu
        275                 280                 285
Gln Lys Ala Ala Glu Glu Ala Ala Arg Val Leu Val Asp Pro Val Pro
    290                 295                 300
Ser Tyr Lys Gln Val Lys Gln Leu Lys Tyr Val Gly Met Val Leu Asn
305                 310                 315                 320
Glu Ala Leu Arg Leu Trp Pro Thr Ala Pro Ala Phe Ser Leu Tyr Ala
                325                 330                 335
Lys Glu Asp Thr Val Leu Gly Gly Glu Tyr Pro Leu Glu Lys Gly Asp
            340                 345                 350
Glu Leu Met Val Leu Ile Pro Gln Leu His Arg Asp Lys Thr Ile Trp
        355                 360                 365
Gly Asp Asp Val Glu Glu Phe Arg Pro Glu Arg Phe Glu Asn Pro Ser
    370                 375                 380
Ala Ile Pro Gln His Ala Phe Lys Pro Phe Gly Asn Gly Gln Arg Ala
385                 390                 395                 400
Cys Ile Gly Gln Gln Phe Ala Leu His Glu Ala Thr Leu Val Leu Gly
                405                 410                 415
Met Met Leu Lys His Phe Asp Phe Glu Asp His Thr Asn Tyr Glu Leu
            420                 425                 430
Asp Ile Lys Glu Thr Leu Thr Leu Lys Pro Glu Gly Phe Val Val Lys
        435                 440                 445
Ala Lys Ser Lys Lys Ile Pro Leu Gly Gly Ile Pro Ser Pro Ser Thr
    450                 455                 460
Glu Gln Ser Ala Lys Lys Val Cys Lys Lys Ala Glu Asn Ala His Asn
465                 470                 475                 480
Thr Pro Leu Leu Val Leu Tyr Gly Ser Asn Met Gly Thr Ala Glu Gly
                485                 490                 495
Thr Ala Arg Asp Leu Ala Asp Ile Ala Met Ser Lys Gly Phe Ala Pro
            500                 505                 510
Gln Val Ala Thr Leu Asp Ser His Ala Gly Asn Leu Pro Arg Glu Gly
        515                 520                 525

Ala Val Leu Ile Val Thr Ala Ser Tyr Asn Gly His Pro Pro Asp Asn
    530                 535                 540
Ala Lys Gln Phe Val Asp Trp Leu Asp Gln Ala Ser Ala Asp Glu Val
545                 550                 555                 560
Lys Gly Val Arg Tyr Ser Val Phe Gly Cys Gly Asp Lys Asn Trp Ala
                565                 570                 575
Thr Thr Tyr Gln Lys Val Pro Ala Phe Ile Asp Glu Thr Leu Ala Ala
            580                 585                 590
Lys Gly Ala Glu Asn Ile Ala Asp Arg Gly Glu Ala Asp Ala Ser Asp
        595                 600                 605
Asp Phe Glu Gly Thr Tyr Glu Glu Trp Arg Glu His Met Trp Ser Asp
    610                 615                 620
Val Ala Ala Tyr Phe Asn Leu Asp Ile Glu Asn Ser Glu Asp Asn Lys
625                 630                 635                 640
Ser Thr Leu Ser Leu Gln Phe Val Asp Ser Ala Ala Asp Met Pro Leu
                645                 650                 655
Ala Lys Met His Gly Ala Phe Ser Thr Asn Val Val Ala Ser Lys Glu
            660                 665                 670
Leu Gln Gln Pro Gly Ser Ala Arg Ser Thr Arg His Leu Glu Ile Glu
        675                 680                 685
Leu Pro Lys Glu Ala Ser Tyr Gln Glu Gly Asp His Leu Gly Val Ile
    690                 695                 700
Pro Arg Asn Tyr Glu Gly Ile Val Asn Arg Val Thr Ala Arg Phe Gly
705                 710                 715                 720
Leu Asp Ala Ser Gln Gln Ile Arg Leu Glu Ala Glu Glu Glu Lys Leu
                725                 730                 735
Ala His Leu Pro Leu Ala Lys Thr Val Ser Val Glu Glu Leu Leu Gln
            740                 745                 750
Tyr Val Glu Leu Gln Asp Pro Val Thr Arg Thr Gln Leu Arg Ala Met
        755                 760                 765
Ala Ala Lys Thr Val Cys Pro Pro His Lys Val Glu Leu Glu Ala Leu
    770                 775                 780
Leu Glu Lys Gln Ala Tyr Lys Glu Gln Val Leu Ala Lys Arg Leu Thr
785                 790                 795                 800
Met Leu Glu Leu Leu Glu Lys Tyr Pro Ala Cys Glu Met Lys Phe Ser
                805                 810                 815
Glu Phe Ile Ala Leu Leu Pro Ser Ile Arg Pro Arg Tyr Tyr Ser Ile
            820                 825                 830
Ser Ser Ser Pro Arg Val Asp Glu Lys Gln Ala Ser Ile Thr Val Ser
        835                 840                 845
Val Val Ser Gly Glu Ala Trp Ser Gly Tyr Gly Glu Tyr Lys Gly Ile
    850                 855                 860
Ala Ser Asn Tyr Leu Ala Glu Leu Gln Glu Gly Asp Thr Ile Thr Cys
865                 870                 875                 880
Phe Ile Ser Thr Pro Gln Ser Glu Phe Thr Leu Pro Lys Asp Pro Glu
                885                 890                 895
Thr Pro Leu Ile Met Val Gly Pro Gly Thr Gly Val Ala Pro Phe Arg
            900                 905                 910
Gly Phe Val Gln Ala Arg Lys Gln Leu Lys Glu Gln Gly Gln Ser Leu
        915                 920                 925
Gly Glu Ala His Leu Tyr Phe Gly Cys Arg Ser Pro His Glu Asp Tyr
    930                 935                 940
Leu Tyr Gln Glu Glu Leu Glu Asn Ala Gln Ser Glu Gly Ile Ile Thr
945                 950                 955                 960
Leu His Thr Ala Phe Ser Arg Met Pro Asn Gln Pro Lys Thr Tyr Val
                965                 970                 975
Gln His Val Met Glu Gln Asp Gly Lys Lys Leu Ile Glu Leu Leu Asp
            980                 985                 990
Gln Gly Ala His Phe Tyr Ile Cys Gly Asp Gly Ser Gln Met Ala Pro
        995                 1000                1005
Ala Val Glu Ala Thr Leu Met Lys Ser Tyr Ala Asp Val His Gln Val
    1010                1015                1020
Ser Glu Ala Asp Ala Arg Leu Trp Leu Gln Gln Leu Glu Glu Lys Gly

1025                    1030                    1035                    1040
Arg Tyr Ala Lys Asp Val Trp Ala Gly
                1045

<210> 7
<211> 1049
<212> PRT
<213> Bakterium megaterium ATCC 145811

<220>
<223> BM3 GVQS

<400> 7
Met Thr Ile Lys Glu Met Pro Gln Pro Lys Thr Phe Gly Glu Leu Lys
1               5                   10                  15
Asn Leu Pro Leu Leu Asn Thr Asp Lys Pro Val Gln Ala Leu Met Lys
            20                  25                  30
Ile Ala Asp Glu Leu Gly Glu Ile Phe Lys Phe Glu Ala Pro Gly Arg
        35                  40                  45
Val Thr Arg Tyr Leu Ser Ser Gln Arg Leu Ile Lys Glu Ala Cys Asp
    50                  55                  60
Glu Ser Arg Phe Asp Lys Asn Leu Ser Gln Gly Leu Lys Phe Val Arg
65                  70                  75                  80
Asp Phe Ala Gly Asp Gly Leu Val Thr Ser Trp Thr His Glu Lys Asn
                85                  90                  95
Trp Lys Lys Ala His Asn Ile Leu Leu Pro Ser Phe Ser Gln Gln Ala
            100                 105                 110
Met Lys Gly Tyr His Ala Met Met Val Asp Ile Ala Val Gln Leu Val
        115                 120                 125
Gln Lys Trp Glu Arg Leu Asn Ala Asp Glu His Ile Glu Val Pro Glu
    130                 135                 140
Asp Met Thr Arg Leu Thr Leu Asp Thr Ile Gly Leu Cys Gly Phe Asn
145                 150                 155                 160
Tyr Arg Phe Asn Ser Phe Tyr Arg Asp Gln Pro His Pro Phe Ile Thr
                165                 170                 175
Ser Met Val Arg Ala Leu Asp Glu Ala Met Asn Lys Gln Gln Arg Ala
            180                 185                 190
Asn Pro Asp Asp Pro Ala Tyr Asp Glu Asn Lys Arg Gln Phe Gln Glu
        195                 200                 205
Asp Ile Lys Val Met Asn Asp Leu Val Asp Lys Ile Ile Ala Asp Arg
    210                 215                 220
Lys Ala Ser Gly Glu Gln Ser Asp Asp Leu Leu Thr His Met Leu Asn
225                 230                 235                 240
Gly Lys Asp Pro Glu Thr Gly Glu Pro Leu Asp Asp Glu Asn Ile Arg
                245                 250                 255
Tyr Gln Ile Ile Thr Phe Leu Ile Ala Gly His Glu Thr Thr Ser Gly
            260                 265                 270
Leu Leu Ser Phe Ala Leu Tyr Phe Leu Val Lys Asn Pro His Val Leu
        275                 280                 285
Gln Lys Ala Ala Glu Glu Ala Ala Arg Val Leu Val Asp Pro Val Pro
    290                 295                 300
Ser Tyr Lys Gln Val Lys Gln Leu Lys Tyr Val Gly Met Val Leu Asn
305                 310                 315                 320
Glu Ala Leu Arg Leu Trp Pro Thr Ala Pro Ser Phe Ser Leu Tyr Ala
                325                 330                 335
Lys Glu Asp Thr Val Leu Gly Gly Glu Tyr Pro Leu Glu Lys Gly Asp
            340                 345                 350
Glu Leu Met Val Leu Ile Pro Gln Leu His Arg Asp Lys Thr Ile Trp
        355                 360                 365
Gly Asp Asp Val Glu Glu Phe Arg Pro Glu Arg Phe Glu Asn Pro Ser
    370                 375                 380
Ala Ile Pro Gln His Ala Phe Lys Pro Phe Gly Asn Gly Gln Arg Ala
385                 390                 395                 400

Cys Ile Gly Gln Gln Phe Ala Leu His Glu Ala Thr Leu Val Leu Gly
                405                 410                 415
Met Met Leu Lys His Phe Asp Phe Glu Asp His Thr Asn Tyr Glu Leu
            420                 425                 430
Asp Ile Lys Glu Thr Leu Thr Leu Lys Pro Glu Gly Phe Val Val Lys
        435                 440                 445
Ala Lys Ser Lys Lys Ile Pro Leu Gly Gly Ile Pro Ser Pro Ser Thr
    450                 455                 460
Glu Gln Ser Ala Lys Lys Val Cys Lys Lys Ala Glu Asn Ala His Asn
465                 470                 475                 480
Thr Pro Leu Leu Val Leu Tyr Gly Ser Asn Met Gly Thr Ala Glu Gly
                485                 490                 495
Thr Ala Arg Asp Leu Ala Asp Ile Ala Met Ser Lys Gly Phe Ala Pro
            500                 505                 510
Gln Val Ala Thr Leu Asp Ser His Ala Gly Asn Leu Pro Arg Glu Gly
        515                 520                 525
Ala Val Leu Ile Val Thr Ala Ser Tyr Asn Gly His Pro Pro Asp Asn
    530                 535                 540
Ala Lys Gln Phe Val Asp Trp Leu Asp Gln Ala Ser Ala Asp Glu Val
545                 550                 555                 560
Lys Gly Val Arg Tyr Ser Val Phe Gly Cys Gly Asp Lys Asn Trp Ala
                565                 570                 575
Thr Thr Tyr Gln Lys Val Pro Ala Phe Ile Asp Glu Thr Leu Ala Ala
            580                 585                 590
Lys Gly Ala Glu Asn Ile Ala Asp Arg Gly Glu Ala Asp Ala Ser Asp
        595                 600                 605
Asp Phe Glu Gly Thr Tyr Glu Glu Trp Arg Glu His Met Trp Ser Asp
    610                 615                 620
Val Ala Ala Tyr Phe Asn Leu Asp Ile Glu Asn Ser Glu Asp Asn Lys
625                 630                 635                 640
Ser Thr Leu Ser Leu Gln Phe Val Asp Ser Ala Ala Asp Met Pro Leu
                645                 650                 655
Ala Lys Met His Gly Ala Phe Ser Thr Asn Val Val Ala Ser Lys Glu
            660                 665                 670
Leu Gln Gln Pro Gly Ser Ala Arg Ser Thr Arg His Leu Glu Ile Glu
        675                 680                 685
Leu Pro Lys Glu Ala Ser Tyr Gln Glu Gly Asp His Leu Gly Val Ile
    690                 695                 700
Pro Arg Asn Tyr Glu Gly Ile Val Asn Arg Val Thr Ala Arg Phe Gly
705                 710                 715                 720
Leu Asp Ala Ser Gln Gln Ile Arg Leu Glu Ala Glu Glu Glu Lys Leu
                725                 730                 735
Ala His Leu Pro Leu Ala Lys Thr Val Ser Val Glu Glu Leu Leu Gln
            740                 745                 750
Tyr Val Glu Leu Gln Asp Pro Val Thr Arg Thr Gln Leu Arg Ala Met
        755                 760                 765
Ala Ala Lys Thr Val Cys Pro Pro His Lys Val Glu Leu Glu Ala Leu
    770                 775                 780
Leu Glu Lys Gln Ala Tyr Lys Glu Gln Val Leu Ala Lys Arg Leu Thr
785                 790                 795                 800
Met Leu Glu Leu Leu Glu Lys Tyr Pro Ala Cys Glu Met Lys Phe Ser
                805                 810                 815
Glu Phe Ile Ala Leu Leu Pro Ser Ile Arg Pro Arg Tyr Tyr Ser Ile
            820                 825                 830
Ser Ser Ser Pro Arg Val Asp Glu Lys Gln Ala Ser Ile Thr Val Ser
        835                 840                 845
Val Val Ser Gly Glu Ala Trp Ser Gly Tyr Gly Glu Tyr Lys Gly Ile
    850                 855                 860
Ala Ser Asn Tyr Leu Ala Glu Leu Gln Glu Gly Asp Thr Ile Thr Cys
865                 870                 875                 880
Phe Ile Ser Thr Pro Gln Ser Glu Phe Thr Leu Pro Lys Asp Pro Glu
                885                 890                 895
Thr Pro Leu Ile Met Val Gly Pro Gly Thr Gly Val Ala Pro Phe Arg

900                 905                 910
Gly Phe Val Gln Ala Arg Lys Gln Leu Lys Glu Gln Gly Gln Ser Leu
        915                 920                 925
Gly Glu Ala His Leu Tyr Phe Gly Cys Arg Ser Pro His Glu Asp Tyr
    930                 935                 940
Leu Tyr Gln Glu Glu Leu Glu Asn Ala Gln Ser Glu Gly Ile Ile Thr
945                 950                 955                 960
Leu His Thr Ala Phe Ser Arg Met Pro Asn Gln Pro Lys Thr Tyr Val
                965                 970                 975
Gln His Val Met Glu Gln Asp Gly Lys Lys Leu Ile Glu Leu Leu Asp
            980                 985                 990
Gln Gly Ala His Phe Tyr Ile Cys Gly Asp Gly Ser Gln Met Ala Pro
        995                 1000                1005
Ala Val Glu Ala Thr Leu Met Lys Ser Tyr Ala Asp Val His Gln Val
    1010                1015                1020
Ser Glu Ala Asp Ala Arg Leu Trp Leu Gln Gln Leu Glu Glu Lys Gly
1025                1030                1035                1040
Arg Tyr Ala Lys Asp Val Trp Ala Gly
                1045

<210> 8
<211> 237
<212> PRT
<213> Mesembryanthemum crystallinum

<220>
<223> McPfOMT

<400> 8
Met Asp Phe Ala Val Met Lys Gln Val Lys Asn Thr Gly Leu Leu Gln
1               5                   10                  15
Ser Glu Glu Leu Cys Gln Tyr Ile Leu Arg Thr Ser Val Tyr Pro Arg
            20                  25                  30
Glu Ala Gly Phe Leu Lys Glu Leu Arg Glu Ala Asn Glu Ser His Pro
        35                  40                  45
Asp Ser Tyr Met Ser Thr Ser Pro Leu Ala Gly Gln Leu Met Ser Phe
    50                  55                  60
Val Leu Lys Leu Val Asn Ala Lys Lys Thr Ile Glu Val Gly Val Phe
65                  70                  75                  80
Thr Gly Tyr Ser Leu Leu Leu Thr Ala Leu Ser Ile Pro Asp Asp Gly
                85                  90                  95
Lys Ile Thr Ala Ile Asp Phe Asp Arg Glu Ala Tyr Glu Ile Gly Leu
            100                 105                 110
Pro Phe Ile Arg Lys Ala Gly Val Glu His Lys Ile Asn Phe Ile Glu
        115                 120                 125
Ser Asp Ala Met Leu Ala Leu Asp Asn Leu Leu Gln Gly Gln Glu Ser
    130                 135                 140
Glu Gly Ser Tyr Asp Phe Gly Phe Val Asp Ala Asp Lys Pro Asn Tyr
145                 150                 155                 160
Ile Lys Tyr His Glu Arg Leu Met Lys Leu Val Lys Val Gly Gly Ile
                165                 170                 175
Val Ala Tyr Asp Asn Thr Leu Trp Gly Gly Thr Val Ala Gln Pro Glu
            180                 185                 190
Ser Glu Val Pro Asp Phe Met Lys Glu Asn Arg Glu Ala Val Ile Glu
        195                 200                 205
Leu Asn Lys Leu Leu Ala Ala Asp Pro Arg Ile Glu Ile Val His Leu
    210                 215                 220
Pro Leu Gly Asp Gly Ile Thr Phe Cys Arg Arg Leu Tyr
225                 230                 235

<210> 9
<211> 363
<212> PRT

<213> Mesembryanthemum crystallinum

<220>
<223> AtCOMT

<400> 9
Met Gly Ser Thr Ala Glu Thr Gln Leu Thr Pro Val Gln Val Thr Asp
1               5                   10                  15
Asp Glu Ala Ala Leu Phe Ala Met Gln Leu Ala Ser Ala Ser Val Leu
            20                  25                  30
Pro Met Ala Leu Lys Ser Ala Leu Glu Leu Asp Leu Leu Glu Ile Met
        35                  40                  45
Ala Lys Asn Gly Ser Pro Met Ser Pro Thr Glu Ile Ala Ser Lys Leu
    50                  55                  60
Pro Thr Lys Asn Pro Glu Ala Pro Val Met Leu Asp Arg Ile Leu Arg
65                  70                  75                  80
Leu Leu Thr Ser Tyr Ser Val Leu Thr Cys Ser Asn Arg Lys Leu Ser
                85                  90                  95
Gly Asp Gly Val Glu Arg Ile Tyr Gly Leu Gly Pro Val Cys Lys Tyr
            100                 105                 110
Leu Thr Lys Asn Glu Asp Gly Val Ser Ile Ala Ala Leu Cys Leu Met
        115                 120                 125
Asn Gln Asp Lys Val Leu Met Glu Ser Trp Tyr His Leu Lys Asp Ala
    130                 135                 140
Ile Leu Asp Gly Gly Ile Pro Phe Asn Lys Ala Tyr Gly Met Ser Ala
145                 150                 155                 160
Phe Glu Tyr His Gly Thr Asp Pro Arg Phe Asn Lys Val Phe Asn Asn
                165                 170                 175
Gly Met Ser Asn His Ser Thr Ile Thr Met Lys Lys Ile Leu Glu Thr
            180                 185                 190
Tyr Lys Gly Phe Glu Gly Leu Thr Ser Leu Val Asp Val Gly Gly Gly
        195                 200                 205
Ile Gly Ala Thr Leu Lys Met Ile Val Ser Lys Tyr Pro Asn Leu Lys
    210                 215                 220
Gly Ile Asn Phe Asp Leu Pro His Val Ile Glu Asp Ala Pro Ser His
225                 230                 235                 240
Pro Gly Ile Glu His Val Gly Gly Asp Met Phe Val Ser Val Pro Lys
                245                 250                 255
Gly Asp Ala Ile Phe Met Lys Trp Ile Cys His Asp Trp Ser Asp Glu
            260                 265                 270
His Cys Val Lys Phe Leu Lys Asn Cys Tyr Glu Ser Leu Pro Glu Asp
        275                 280                 285
Gly Lys Val Ile Leu Ala Glu Cys Ile Leu Pro Glu Thr Pro Asp Ser
    290                 295                 300
Ser Leu Ser Thr Lys Gln Val Val His Val Asp Cys Ile Met Leu Ala
305                 310                 315                 320
His Asn Pro Gly Gly Lys Glu Arg Thr Glu Lys Glu Phe Glu Ala Leu
                325                 330                 335
Ala Lys Ala Ser Gly Phe Lys Gly Ile Lys Val Val Cys Asp Ala Phe
            340                 345                 350
Gly Val Asn Leu Ile Glu Leu Leu Lys Lys Leu
        355                 360

<210> 10
<211> 232
<212> PRT
<213> Mesembryanthemum crystallinum

<220>
<223> AOMT

<400> 10
Met Ala Lys Asp Glu Ala Lys Gly Leu Leu Lys Ser Glu Glu Leu Tyr

1               5                   10                  15
Lys Tyr Ile Leu Glu Thr Ser Val Tyr Pro Arg Glu Pro Glu Val Leu
            20                  25                  30
Arg Glu Leu Arg Asn Ile Thr His Asn His Pro Gln Ala Gly Met Ala
        35                  40                  45
Thr Ala Pro Asp Ala Gly Gln Leu Met Gly Met Leu Leu Asn Leu Val
    50                  55                  60
Asn Ala Arg Lys Thr Ile Glu Val Gly Val Phe Thr Gly Tyr Ser Leu
65                  70                  75                  80
Leu Leu Thr Ala Leu Thr Leu Pro Glu Asp Gly Lys Val Ile Ala Ile
                85                  90                  95
Asp Met Asn Arg Asp Ser Tyr Glu Ile Gly Leu Pro Val Ile Lys Lys
            100                 105                 110
Ala Gly Val Glu His Lys Ile Asp Phe Lys Glu Ser Glu Ala Leu Pro
        115                 120                 125
Ala Leu Asp Glu Leu Leu Asn Asn Lys Val Asn Glu Gly Gly Phe Asp
    130                 135                 140
Phe Ala Phe Val Asp Ala Asp Lys Leu Asn Tyr Trp Asn Tyr His Glu
145                 150                 155                 160
Arg Leu Ile Arg Leu Ile Lys Val Gly Gly Ile Ile Val Tyr Asp Asn
                165                 170                 175
Thr Leu Trp Gly Gly Ser Val Ala Glu Pro Asp Ser Ser Thr Pro Glu
            180                 185                 190
Trp Arg Ile Glu Val Lys Lys Ala Thr Leu Glu Leu Asn Lys Lys Leu
        195                 200                 205
Ser Ala Asp Gln Arg Val Gln Ile Ser Gln Ala Ala Leu Gly Asp Gly
    210                 215                 220
Ile Thr Ile Cys Arg Arg Leu Tyr
225                 230

<210> 11
<211> 43
<212> DNA
<213> Bacillus megaterium

<220>
<223> Primersequenz

<400> 11
ttgataaaaa cttaagtcaa ggtcttaaat ttgtacgtga ttt                         43


<210> 12
<211> 43
<212> DNA
<213> Bacillus megaterium

<220>
<223> Primersequenz

<400> 12
aaatcacgta caaatttaag accttgactt aagtttttat caa                         43


<210> 13
<211> 43
<212> DNA
<213> Bacillus megaterium

<220>
<223> Primersequenz

<400> 13

tggatgaagc aatgaacaag cagcagcgag caaatccaga cga 43

<210> 14
<211> 43
<212> DNA
<213> Bacillus megaterium

<220>
<223> Primersequenz

<400> 14
tcgtctggat ttgctcgctg ctgcttgttc attgcttcat cca 43

<210> 15
<211> 30
<212> DNA
<213> Bacillus megaterium

<220>
<223> Primersequenz

<400> 15
tatggccaac tgctccttcg ttttccctat 30

<210> 16
<211> 30
<212> DNA
<213> Bacillus megaterium

<220>
<223> Primersequenz

<400> 16
atagggaaaa cgaaggagca gttggccata 30

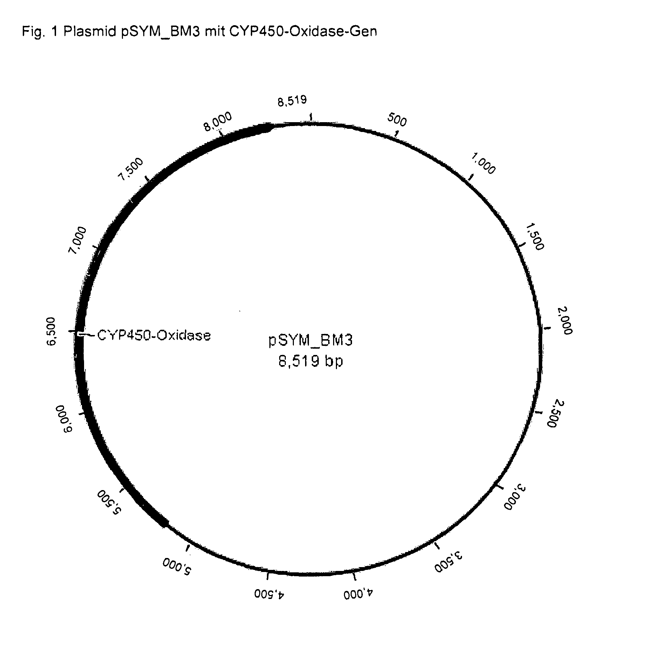

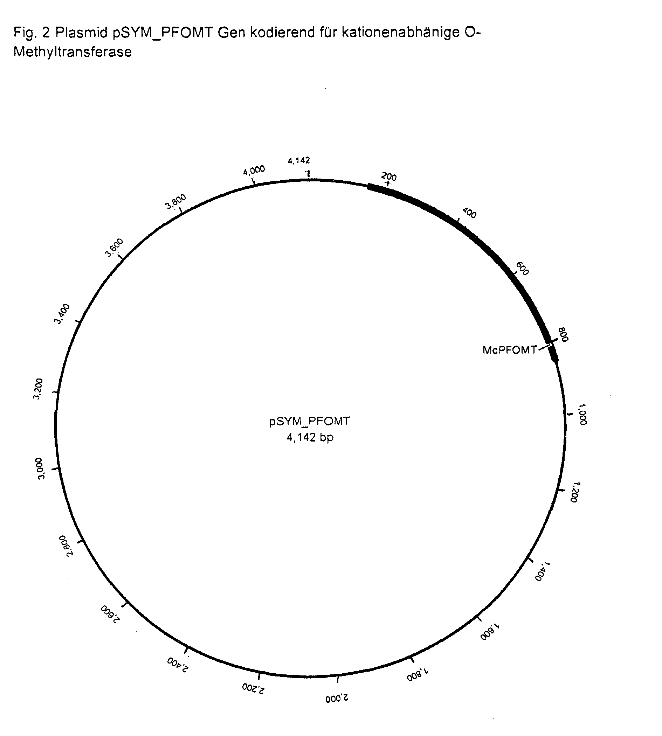

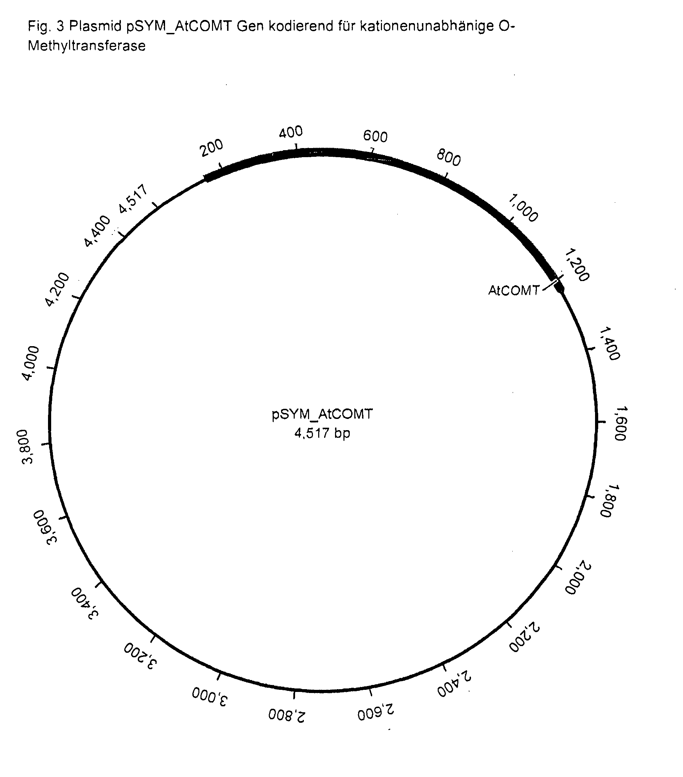

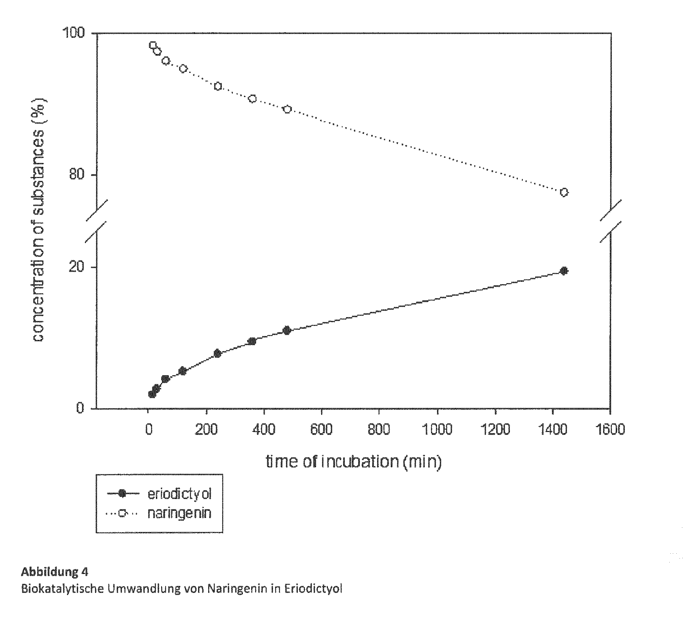

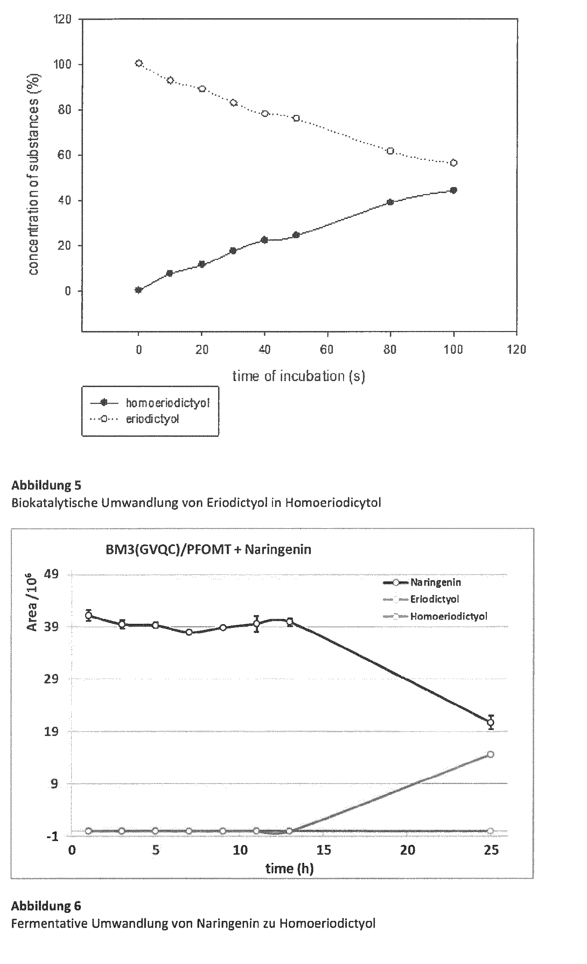

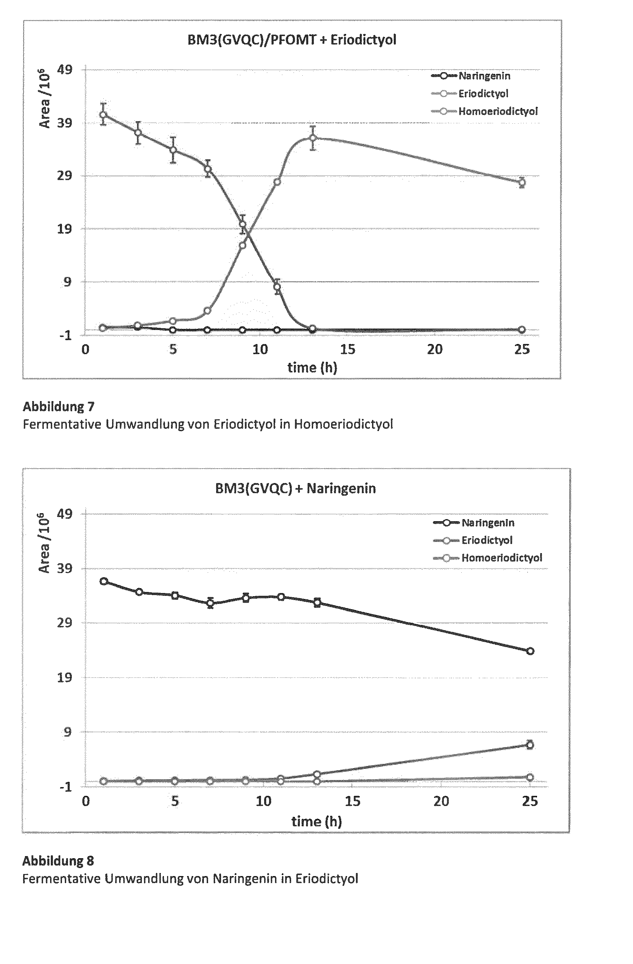

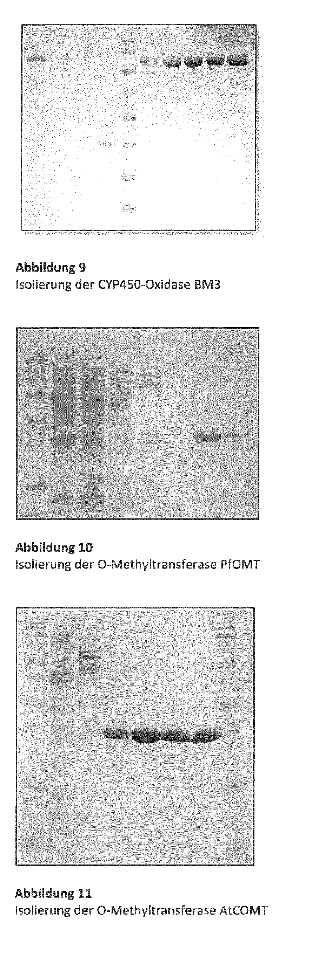

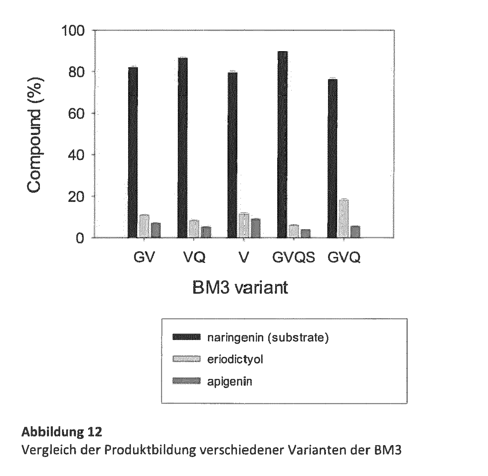

## Patentansprüche

1. Verfahren zur Herstellung von Flavonoiden, umfassend die Schritte:
(a) Bereitstellen eines transgenen Mikroorganismus, enthaltend
(i) einen ersten Nukleinsäure-Abschnitt (A), umfassend oder bestehend aus einem für eine CYP450-Oxidase kodierenden Gen,
(ii) einen zweiten Nukleinsäure-Abschnitt (B), umfassend oder bestehend aus einem für eine O-Methyltransferase kodierenden Gen, sowie
(b) Hinzufügen eines oder mehrerer Flavanone zu dem transgenen Mikroorganismus,
(c) Kultivierung des transgenen Mikroorganismus unter Bedingungen, die die Umsetzung der Flavanone zu den entsprechenden Flavonoiden ermöglichen, sowie gegebenenfalls
(d) Isolierung und Aufreinigung der Endprodukte.

2. Verfahren nach Anspruch 1, **dadurch gekennzeichnet, dass** das für eine bakterielle CYP450 Oxidase kodierende Gen für eine Oxidase aus dem Mikrorganismus *Bacillus megaterium* kodiert.

3. Verfahren nach den Ansprüchen 1 und/oder 2, **dadurch gekennzeichnet, dass** das für eine pflanzliche O-Methyltransferase kodierende Gen für eine O-Methyltransferase aus der Pflanzengattung *Mesembryanthemum* kodiert.

4. Verfahren nach mindestens einem der Ansprüche 1 bis 3, **dadurch gekennzeichnet dass** der in den transgenen Mikroorganismus eingebrachte Nukleinsäure-Abschnitt (A)
(i-i) eine Nukleotidsequenz gemäß **SEQ ID NO:1** (Nukleotidsequenz der für die A75G/ F88V/L189Q/R472C Variante der bakterielle CYP450 aus *B*. *megaterium* ATCC 14581 kodierenden Gens) und/oder
(i-ii) eine Nukleotidsequenz **SEQ ID NO: 2** (Nukleotidsequenz der für die A75G/ F88V/L189Q/A331S/R472C Variante der bakterielle CYP450 aus *B*. *megaterium* ATCC 14581 kodierenden Gens) und/oder
(i-iii) eine ähnliche Nukleotidsequenz mit einer Nukleotidsequenz-Identität von 40 % oder mehr zu **SEQ ID NO:1** und/oder **SEQ ID NO:2**, vorzugsweise von 50 % oder mehr, 60 % oder mehr oder 80 % oder mehr, besonders bevorzugt von 95 % oder mehr,
umfasst oder daraus besteht.

5. Verfahren nach mindestens einem der Ansprüche 1 bis 4, **dadurch gekennzeichnet, dass** die CYP450 Oxidase
(i-i) eine Nukleotidsequenz gemäß **SEQ ID NO:1** (Nukleotidsequenz der für die A75G/ F88V/L189Q/R472C Variante der bakterielle CYP450 aus *B. megaterium* ATCC 14581 kodierenden Gens) und/oder
(i-ii) eine Nukleotidsequenz **SEQ ID NO:2** (Nukleotidsequenz der für die A75G/ F88V/L189Q/A331S/R472C Variante der bakterielle CYP450 aus *B. megaterium* ATCC 14581 kodierenden Gens) und/oder
(i-iii) eine ähnliche Nukleotidsequenz mit einer Nukleotidsequenz-Identität von 40 % oder mehr zu **SEQ ID NO:1** und/oder **SEQ ID NO:2**, vorzugsweise von 50 % oder mehr, 60 % oder mehr oder 80 % oder mehr, besonders bevorzugt von 95 % oder mehr,
enthält.

6. Verfahren nach mindestens einem der Ansprüche 1 bis 5, **dadurch gekennzeichnet, dass** der in den transgenen Mikroorganismus eingebrachte Aminosäure-Abschnitt (A)
(i-i) eine Aminosäuresequenz gemäß **SEQ ID NO:6** (Aminosäuresequenz der für die A75G/ F88V/L189Q/R472C Variante der bakterielle CYP450 aus *B. megaterium* ATCC 14581 kodierenden Gens) und/oder
(i-ii) eine Aminosäuresequenz **SEQ ID NO:7** (Aminosäuresequenz der für die A75G/ F88V/L189Q/A331S/R472C Variante der bakterielle CYP450 aus *B. megaterium* ATCC 14581 kodierenden Gens) und/oder
(i-iii) eine ähnliche Aminosäuresequenz mit einer Aminosäuresequenz-Identität von 40 % oder mehr zu **SEQ ID NO:6** und/oder **SEQ ID NO:7**, vorzugsweise von 50 % oder mehr, 60 % oder mehr oder 80 % oder mehr, besonders bevorzugt von 95 % oder mehr,
umfasst oder daraus besteht.

7. Verfahren nach mindestens einem der Ansprüche 1 bis 6, **dadurch gekennzeichnet, dass** die vorzugsweise bakterielle CYP450 Oxidase
(i-i) eine Aminosäuresequenz gemäß **SEQ ID NO:6** (Aminosäuresequenz der für die A75G/ F88V/L189Q/R472C Variante der bakterielle CYP450 aus *B*. *megaterium* ATCC 14581 kodierenden Gens) und/oder
(i-ii) eine Aminosäuresequenz **SEQ ID NO:7** (Aminosäuresequenz der für die A75G/ F88V/L189Q/A331S/R472C Variante der bakterielle CYP450 aus *B*. *megaterium* ATCC 14581 kodierenden Gens) und/oder
(i-iii) eine ähnliche Aminosäuresequenz mit einer Aminosäuresequenz-Identität von 40 % oder mehr zu **SEQ ID NO:6** und/oder **SEQ ID NO:7**, vorzugsweise von 50 % oder mehr, 60 % oder mehr oder 80 % oder mehr, besonders bevorzugt von 95 % oder mehr,
enthält.

8. Verfahren nach mindestens einem der Ansprüche 1 bis 7, **dadurch gekennzeichnet dass** der in den transgenen Mikroorganismus eingebrachte Nukleinsäure-Abschnitt (B)
(ii-i) eine Nukleotidsequenz gemäß **SEQ ID NO:3** (Nukleotidsequenz der OMT aus M. *crystallinum* (McPFOMT)), und/oder
(ii-ii) eine Nukleotidsequenz gemäß **SEQ ID NO:4** (Nukleotidsequenz der O-Methyltransferase 1 (OMT1)) und/oder
(ii-iii) eine Nukleotidsequenz gemäß **SEQ ID NO:5** (Nukleotidsequenz der O-Methyltransferase (AOMT)) und/oder
(ii-iv) eine weitere Nukleotidsequenz mit einer Nukleotidsequenz-Identität von 40 % oder mehr zu **SEQ ID NO:3, SEQ ID NO:4 und/oder SEQ ID NO:5**, vorzugsweise von 50 % oder mehr, 60 % oder mehr oder 80 % oder mehr, besonders bevorzugt von 95 % oder mehr,
umfasst oder daraus besteht.

9. Verfahren nach mindestens einem der Ansprüche 1 bis 8, **dadurch gekennzeichnet, dass** die O-Methyltransferase
(ii-i) eine Nukleotidsequenz gemäß **SEQ ID NO:3** (Nukleotidsequenz der OMT aus M. *crystallinum* (McPFOMT)), und/oder
(ii-ii) eine Nukleotidsequenz gemäß **SEQ ID NO:4** (Nukleotidsequenz der O-Methyltransferase 1 (OMT1)) und/oder
(ii-iii) eine Nukleotidsequenz gemäß **SEQ ID NO:5** (Nukleotidsequenz der O-Methyltransferase (AOMT)) und/oder
(ii-iv) eine weitere Nukleotidsequenz mit einer Nukleotidsequenz-Identität von 40 % oder mehr zu **SEQ ID NO:3, SEQ ID NO:4 und/oderSEQ ID NO:5**, vorzugsweise von 50 % oder mehr, 60 % oder mehr oder 80 % oder mehr, besonders bevorzugt von 95 % oder mehr,
enthält.

10. Verfahren nach mindestens einem der Ansprüche 1 bis 9, **dadurch gekennzeichnet, dass** der in den transgenen Mikroorganismus eingebrachte Aminosäure-Abschnitt (B) (ii-i) eine Aminosäuresequenz gemäß **SEQ ID NO:8** (Aminosäuresequenz der OMT aus M. *crystallinum* (McPFOMT)), und/oder
(ii-ii) eine Aminosäuresequenz gemäß **SEQ ID NO:9** (Aminosäuresequenz der O-Methyltransferase 1 (OMT1)) und/oder
(ii-iii) eine Aminosäuresequenz gemäß **SEQ ID NO:10** (Aminosäuresequenz der O-Methyltransferase (AOMT)) und/oder
(ii-iv) eine weitere Aminosäuresequenz mit einer Aminosäuresequenz-Identität von 40 % oder mehr zu **SEQ ID NO:8, SEQ ID NO:8** und/oder **SEQ ID NO:10**, vorzugsweise von 50 % oder mehr, 60 % oder mehr oder 80 % oder mehr, besonders bevorzugt von 95 % oder mehr,
umfasst oder daraus besteht.

11. Verfahren nach mindestens einem der Ansprüche 1 bis 10, **dadurch gekennzeichnet, dass** die O-Methyltransferase
(ii-i) eine Aminosäuresequenz gemäß **SEQ ID NO:8** (Aminosäuresequenz der OMT aus M. *crystallinum* (McPFOMT)), und/oder
(ii-ii) eine Aminosäuresequenz gemäß **SEQ ID NO:9** (Aminosäuresequenz der O-Methyltransferase 1 (OMT1)) und/oder
(ii-iii) eine Aminosäuresequenz gemäß **SEQ ID NO:10** (Aminosäuresequenz der O-Methyltransferase (AOMT)) und/oder
(ii-iv) eine weitere Aminosäuresequenz mit einer Aminosäuresequenz-Identität von 40 % oder mehr zu **SEQ ID NO:8, SEQ ID NO:9** und/oder **SEQ ID NO:10**, vorzugsweise von 50 % oder mehr, 60 % oder mehr oder 80 % oder mehr, besonders bevorzugt von 95 % oder mehr,
enthält.

12. Verfahren nach mindestens einem der Ansprüche 1 bis 11, **dadurch gekennzeichnet, dass** die Flavanone ausgewählt sind aus der Gruppe, die gebildet wird von Naringenin, Naringin, Narirutin, oder anderen natürlich vorkommenden oder künstlich hergestellten Naringeninglycosiden, Sakuranetin, Sakuranetinglycoside, Isosakuranetin, Isosakuranetinglycosiden, 4',7-Dihydroxy-flavanon oder dessen Glycosiden, 4',7-Dihydroxy-3'-methoxy-flavanon oder dessen Glycosiden, 3',7-Dihydroxy-4'-methoxy-flavanon oder dessen Glycosiden, 3',4',7-Trihydroxy-flavanon oder dessen Glycosiden, wobei die Flavanone bezüglich der 2-Position des Flavanongerüsts als (S)-, als (R)-Enantiomer, als Racemat oder als beliebige Mischung der beiden Enantiomere vorliegen können.

13. Transgener Mikroorganismus, enthaltend
(i) einen ersten Nukleinsäure-Abschnitt (A) enthaltend ein für eine bakterielle CYP450 Oxidase kodierendes Gen, sowie
(ii) einen zweiten Nukleinsäure-Abschnitt (B), enthaltend ein für eine pflanzliche O-Methyltransferase kodierendes Gen.

14. Mikroorganismus nach Anspruch 13, **dadurch gekennzeichnet, dass** es sich um einen fakultativ anaeroben Mikroorganismus handelt.

15. Mikroorganismus nach den Ansprüchen 13 und/oder 14, **dadurch gekennzeichnet, dass**
(i) das für eine CYP450-Oxidase kodierende Gen für eine CYP450-Oxidase aus dem Mikroorganismus *Bacillus megaterium* (BM3) kodiert, und/oder
(ii) das für eine O-Methyltransferase kodierende Gen für eine O-Methyltransferase aus *A. thaliana* oder M. *crystalliunum* kodiert.

16. Vektor, enthaltend
(i) einen ersten Nukleinsäure-Abschnitt (A) enthaltend ein für eine bakterielle CYP450 Oxidase kodierendes Gen, sowie
(ii) einen zweiten Nukleinsäure-Abschnitt (B), enthaltend ein für eine pflanzliche O-Methyltransferase kodierendes Gen.

17. Vektor nach Anspruch 16, d**adurch gekennzeichnet**, dass er
(i) einen ersten Nukleinsäure-Abschnitt (A) enthaltend ein für eine bakterielle CYP450 Oxidase kodierendes Gen, sowie
(ii) einen zweiten Nukleinsäure-Abschnitt (B), enthaltend ein für eine pflanzliche O-Methyltransferase kodierendes Gen.
aufweist.

18. Wirtszelle, enthaltend mindestens einen Vektor nach den Ansprüchen 16 und/oder 17.

19. Nahrungsmittel, enthaltend Flavonoide, erhalten nach dem Verfahren nach mindestens einem der Ansprüche 1 bis 12.
